Europäisches Patentamt

European Patent Office

Office européen des brevets

Publication number: **0 273 371**
**A2**

# EUROPEAN PATENT APPLICATION

(21) Application number: **87119065.8**

(22) Date of filing: **22.12.87**

(51) Int. Cl.⁴: **A22B 5/00 , A22B 7/00**

(30) Priority: **23.12.86 DK 6295/86**
**26.06.87 DK 3292/87**

(43) Date of publication of application:
**06.07.88 Bulletin 88/27**

(84) Designated Contracting States:
**AT BE CH DE ES FR GB IT LI NL SE**

(71) Applicant: **Slagteriernes Forskningsinstitut**
**Maglegaardsvej 2**
**DK-4000 Roskilde(DK)**

(72) Inventor: **Madsen, K.B.**
**Baldersvej 19**
**DK-4000 Roskilde(DK)**
Inventor: **Post, Erling**
**Bybakken 14 Annisse**
**DK-3200 Helsinge(DK)**
Inventor: **Christensen, Henning**
**Kongebakken 32**
**DK-4000 Roskilde(DK)**
Inventor: **Halden, Steen**
**Davidsvaenge 23**
**DK-3480 Fredensborg(DK)**
Inventor: **Kristensen, Jorgen Korsgaard**
**Laerkevej 12**
**DK-2630 Tastrup(DK)**

(74) Representative: **Patentanwälte Dipl.-Ing. Bodo**
**Thielking Dipl.-Ing. Otto Elbertzhagen**
**Gadderbaumer Strasse 20**
**D-4800 Bielefeld 1(DE)**

(54) **A method and apparatus for treatment or examination of carcasses.**

(57) In a method for treatment or examination of carcasses of slaughter animals, especially hogs (39, 40, 46) in a treatment or examination station the carcasses are forwarded consecutively in a continuously working first transport system in which the carcasses, suspended by their hind legs, are forwarded along a given path and at a rate of typically 200-400 carcasses per hour. From this first transport system the carcasses are transferred to an intermittently working second transport system in which the individual carcass (40) is forwarded, suspended from its hind legs and held in a fixation frame (17) which is circulated in a closed path. Before the transfer to a frame the total length of the carcass is measured in a station (49). On the basis of this measurement of length the frame is positioned correctly in relation to the carcass by means of a device (81) for height adjustment of the head fastening means of the frame. In a subsequent station the position of given anatomical positions, foreleg and collarbone, of the individual carcass is determined, and on the basis of said total length and these further position determinations insertion probes are controlled, which probes are correctly positioned in relation to the carcass, and which are inserted at desired places in the carcass, and by means of which a quality determination is made, on the basis of which quality determination a control of branding assemblies (86, 87, 88, 89 and 90) is performed. After branding, the carcass is transferred from the second, intermittently working transport system to the first, continuously working transport system.

On the basis of the measurement of length and the position determinations made, the probes and

the branding assemblies and any other treatment means such as sampling means or the like are controlled. On the basis of the quality determination the carcass may be not only branded, but also classified appropriately, as the various parts of the carcass may be exploited in the best possible manner on the basis of the quality determination made. All these data concerning length, positions, quality, classification, etc., are stored and processed in a central computer (10, 11).

Fig. 1

## A METHOD AND APPARATUS FOR TREATMENT OR EXAMINATION OF CARCASSES

The present invention relates to a method for treatment or examination of carcasses of slaughter animals, especially hogs or porkers, in at least one treatment or examination station, in which method the carcasses are moved substantially continuously and consecutively by a first transport system, such as a sliding rail transport system, along a given path, the carcasses being passed to and from the treatment or examination station by the first transport system, and in which method the treatment or the examination is carried out in the treatment or examination station by means of treatment or examination means adjustable relative to the individual carcass.

Since about 1975 practically all Danish bacon factories have employed so-called KSA meters (KSA: in Danish: Køs/Spæk/Automatik; in English: Meat/Lard/Automatics) for determination of quality and classification of slaughtered hogs. These KSA meters are meters operated manually or by an operator which meters are introduced by the operator at predetermined places in a carcass which is being passed by the transport system along a given path in a slaughtering hall of a slaughterhouse or bacon factory. In this manually performed classification, an automatic registration of the thickness of the lard and meat is made for each introduction into the carcass of the measuring probe of the KSA meter. On the basis of the registrations made and the weight of the carcass a computer makes a calculation of the sales class of the carcass in question, which class is displayed on a display on the KSA meter. By branding on predetermined places of the carcass, the sales class to which the carcass in question belongs is marked. This known manual method of classification has, however, certain limitations as the time available for carrying out the classification method for each carcass is determined by the production rate of the slaughter house, which, for a single conveyor may be of the order of 200 to 400 carcasses per hour, typically about 300, which means that there are only a few seconds available to carry out this manual classification. Consequently it is understood that the classification method can only comprise quite few measuring points on the individual carcass, and that a more finely specifying classification, for example of various parts of the individual carcass, is not possible as a result of the short time available to carry out the whole classification method.

Besides this classification of the carcass, normally a large number of other manual operations are also made to the carcasses, including branding, cutting open, etc.

With a view to automating several of these operations, especially the cutting open, but also the determination of quality by the introduction of measuring probes into the carcasses at predetermined locations thereof, it has been proposed, vide the specification of Danish patent application No. 330/82, to control the means, by the aid of which the treatment or examination in question is made, especially the cutting open, the determination of quality by means of probes, etc., by transferring the individual carcass which is passed along by said first transport system from said first transport system to a roundabout in a feeder station thereof, on which roundabout the carcass is maintained in a fixture, whereupon the roundabout is turned from this feeder station to a measuring station in which an automatic measuring of predetermined parts of the carcass is made by means of measuring means. Then the roundabout is again turned 90° to a first one of two cutting open station in which the carcass is cut open. This patent application states that apart from this cutting open of the carcass in one of two cutting open stations, a quality determination may be made by introducing measuring probes in dependency of the automatic measuring made of predetermined parts of the carcass, but any detailed explanation of how and where these measuring probes might be arranged, and how the measuring probes might function is not given in this patent application. In this connection it should be considered that a transport system with which such a roundabout must cooperate in consequence of the above high rate of production sets very narrow time limites to the operations which may be carried out at the individual stations. Furthermore, the whole roundabout structure becomes very heavy as the apparatus is limited to four stations, a feeding station, a measuring station and two cutting open stations, which restrict the kind, number and complexity of the operations, including cutting open operations, treatments and examinations, which may be carried out on the individual carcass in the three "active" stations. Also, this structure renders it extremely difficult to correct defects, if any, without having to stop the first transport system, and in practice it has also proved difficult to obtain a safe and reliable feeding of the individual carcass into the fixture of the roundabout at the above high rate of production. The roundabout structure itself will further cause substantial difficulties, such as indicated above, as the structure necessarily must be rather strong and thus becomes heavy, and as, on one hand, the turning from one station to the next station must take place quickly, which causes considerable problems relative to transfer of the forces and moments necessary for the quick turning in connection with a strong and thus heavy roundabout structure, and on the other hand must take place to suitably slowly that the carcasses arranged in the fixtures of the roundabout do not change their position in the fixtures of perhaps are ejected therefrom as a result of the centrifugal force.

There is thus a need for a method of the type described above in which method the desired treatment or examination of the carcasses may be made safely and reliably at a production rate of for example of the order of 200 to 400 carcasses per houd, and which method renders it possible to make any number of treatments or examinations.

This purpose is obtained in accordance with the present invention in that the carcasses are passed through the treatment or examination station by means of a second transport system which is an intermittently working transport system and which defines a substantially linear transportation path, at which the treatment or examination means are arranged, in that the carcasses are transferred from the first transport system to the second transport system prior to the treatment or examination station, and in that after transportation through the treatment or examination station by means of this second tranpsort system the carcasses are transferred therefrom to the first transport system.

By the provision of this second transport system as a transport system working intermittently and defining a substantially linear transportation path, it is possible to arrange any number of treatment or examination stations at this second transport system in which stations the duration of the treatment or examination carried out in the individual station must not exceed the period determined by the rate of transportation of the first, substantially continuously working transport system. If a treatment or an examination takes more time than permitted by the period defined by the first transport system, it will therefor be possible to divide the treatment or examination into part operations and then carry out the part operations at individual stations without having to consider that the number of stations must not exceed a given maximum. As described above, the first, substantially continuously working transport system of a bacon factory is capable of transporting about 200 to 400 carcasses per hour. The requirement to the execution of the part operation at the individual station is thus only that it must be possible to carry out the part operation in a maximum of about 10 seconds. By the provision of a substantially linear transportation path of the second, intermittently working transport system the advancing of the carcasses through the treatment or examination station becomes far less complicated than in the roundabout apparatus described above, and the possible risk of dropping or destroying a carcass or of a carcass blocking the transport system is significantly smaller than in this known roundabout apparatus.

The invention also relates to an apparatus for treatment or examination of carcasses of slaughter animals, especially hogs, and comprises at least one treatment or examination station and a first transport system in which the carcasses are passed substantially continuously and consecutively along a given path, the carcasses being passed to and from the treatment or examination station by the first transport system, at which treatment or examination station the treatment or examination is carried out by means of treatment or examination means adjustable relative to the individual carcass and the apparatus according to the invention, by means of which the above objects may be obtained, is characterized in that the apparatus comprises a second transport system for moving the carcasses through the treatment or examination station, that the second transport system is an intermittently working transport system defining a substantially linear path of transportation at which the treatment or examination means are arranged, and that the first and the second transport systems are adapted to transfer the individual carcass from the first transport system to the second transport system prior to the treatment or examination station and to transfer the individual carcass from the second transport system to the first transport system after transportation through the treatment or examination station.

According to the preferred embodiment of the method and the apparatus of the invention the carcasses are transferred from the first transport system to an input station arranged in the second transport system prior to the treatment or examination station, and a registration of a measure of at least one outer dimension of the individual carcass is made prior to or at the input station and/or at a registration station arranged between the input station and the treatment or examination station. On the basis of empirical data for the kind of slaughter animal in question this measure is converted into a control signal for controlling the positioning of the treatment or examination means relative to the carcass in question.

The slaugher animals to be treated or examined are typically cows, heifers, steers, calves, sheep, but preferably hogs. In connection with transportation of such slaughter animals, especially hogs, in a transport system such as the first, substantially continuously working transport system, the carcasses are usually passed along suspended in so-called gambrels, suspended by their legs. In accordance with a further embodiment of the method and apparatus of the invention the carcasses are therefore passed along the first and second transport systems suspended by their legs. In accordance with this further embodiment of the method and the apparatus of the present invention of the registration of the measure of at least said one outer dimension of the individual carcass at the input station is made by registration of a measure of the total length of the individual carcass, the registration of this measure preferably being made by means of a movable plate comprising an associated position detecting device, the movable plate being adapted to be brought into contact with the bottommost part of the individual carcass, i.e. the snout.

Besides registration of the total length of the individual carcass a registration is preferably made of a measure representing the positioning of at least one extremity of the individual carcass, especially the positioning of one foreleg, and preferably a further registration of a measure representing the positioning of the collarbone or clavicle of the individual carcass. In practice, in connection with the treatment and examination of hogs it has proved that the provision of these three measures of the individual carcass, viz. the total length of the carcass, the positioning of an extremity, especially the foreleg, and the positioning of the collarbone, yields a fully sufficient representation of the individual carcass for the treatment or examination means, which are employed in the treatment or examination station for carrying out the desired treatment or examination, to be positioned correctly and to carry out the desired treatment or examination within the period determined by the rate of transportation of the transport system.

The conversion of said measure or measures into said control signal may be made in any manner in accordance with the relevant kind of slaughter animal by using an arithmetic algorithm, but the conversion is advantageously made in accordance with a linear algorithm representing the positions of said extremity and the collarbone relative to the total length of the carcass.

With a view to classification of the individual carcass it is preferred that a registration is further made of the weight of the individual slaughter animal. The registration of the weight of the individual slaughter animal may be made by means of separate scales or may be provided from the weighing-in station of the slaughter house where a registration of the weight of the individual slaughter animal is made already on reception of the slaughter animals.

The treatment or examination carried out in the treatment or examination station is preferably a test sampling, quality testing, marking or cutting up. By test sampling the treatment or examination station contains test sampling means, i.e. means comprising a sampling probe, which removes a sample of a carcass by insertion into and subsequent extraction from the carcass. Such sampling means may, in connection with hogs, preferably be used for determination of any boar smell. The quality testing means may be of any type and may typically comprise an insertion probe, which determines the thickness of the lard layers and skin layers by means of optical measurement and registration of the depth of insertion or introduction, or be an electric conductivity meter, an interface detecting device comprising an ultra sound emitter and an ultra sound receiver or any other type of quality testing means. These quality testing means are, however, preferably of the type described in Danish patent application No. 4247/86. The cutting up means may be of any type and comprise cutting or sawing devices, while the marking means may comprise any classification stamps, veterinary stamps, stamps for identification or origin and date.

The first transport system will in most cases be a transport system which already exists in a slaughter house, especially a transport system in whcih the carcasses are passed along suspended by their hind legs in gambrels. The second transport system firstly has to be adapted to the first transport system in such a manner that the transfer of the individual carcass from the first transport system to the second transport system and back to the first transport system becomes as simple as possible and may be carried out in a reliable and safe manner. Further the second transport system has to satisfy any requirements made by the treatment or examination station with regard to fixation of the individual carcass, and may be a transport system with an endless conveyor, for example a belt conveyor, a chain conveyor or the like. It is preferred that in the second transport system the carcasses are passed along suspended in frames for fixation of the caracasses, and that the frames are passed along in a closed path of which said substantially linear path of transportation constitutes a part. By forwarding the carcasses in the frame in the second transport system a secure fixation of the carcasses is obtained when registration of said measure or measures is carried out, and further when the carcasses are presented to said treatment or examination means at the treatment or examination station. As the carcasses do not need to be fixed during transportation in the first transport system, substantial savings are obtained by the carcasses being forwarded fixed in frames only in the second transport system as, in connection with large slaughter houses, only a limited number of frames for fixation of the carcasses is needed while the carcasses are passed through the treatment station or substation, and as the frames are immediately re-used after being returned from the output station of the second transport system to the input station thereof.

In accordance with a further embodiment of the apparatus according to the invention, the frames are suspended in a guide rail defining the closed path, and the frames are further preferably adapted to be guided in an underlying pilot rail defining the same closed path as the guide rail from which the frames are suspended. This guiding of the frames in a suspension rail and an underlying pilot rail yields a particularly safe and reliable guiding of the frame and thus of the carcasses arranged on the frames through the treatment or examination station. Instead of guiding the frames in a suspension rail and an underlying pilot rail the frames may, in accordance with alternative embodiments of the apparatus of the invention, be guided in one or more roller and bearing rails, just as the second transport system of the apparatus may be of any suitable type, for example a closed belt conveyor with suitable carriers constituting the fixation frames described above.

In the embodiment of the apparatus of the invention described above, in which embodiment the frames are adapted to be guided in a closed path, the second transport system of the apparatus is preferably adapted to pass along the frames in the part of the closed path which does not constitute said substantially linear path, in a continuous movement from an output station at which the carcasses are transferred from the second transport system to the first transport system, to said input station. This continuous return of the frames from the output station to the input station is preferably provided by means of a continuously working conveyor, the frames and the conveyor being adapted to connect the frames to the conveyor at the output station and to disconnect the frames from the conveyor at the input station. The intermittent forwarding of the carcasses arranged on the frames through the treatment or examination station may, in accordance with the preferred embodiment of the apparatus of the invention, be provided by the second transport system of the apparatus having a forwarding mechanism which may be connected with and disconnected from at least one frame and which is adapted to be driven by a fluid drive mechanism, i.e. a hydraulic or pneumatic drive mechanims for providing the intermittent forwarding of a frame connected with the forwarding mechanism, the forwarding mechanism, which may be connected to and disconnected from a frame, preferably being a gripping mechanism which may be connected with and disconnected from one frame. The above continuously working conveyro is preferably a chain conveyor having carrier members which are adapted to provide the connection of the frames to the conveyor at the output station and the disconnection of the frames from the conveyor at the input station, the carrier members preferably being constituted by hinged carrier fingers.

To obtain a safe and reliable transfer of a carcass from the first transport system to the second transport system at the input station, and to obtain a safe and reliable transfer of a carcass from the second transport system to the first transport system at the output station, the apparatus preferably comprises gripping means adapted to transfer a carcass from the first transport system to the second transport system and to arrange the carcass on a frame, and ejector means adapted to eject a carcass from its associated frame and to transfer the carcass from the second transport system to the first transport system.

The frame is preferably an openable frame, the apparatus comprising means at the input and output stations for opening the frames for introduction of a carcass into a frame and for ejection of a carcass from a frame, respectively, the openable frame preferably comprising gripping members for gripping around the head of a carcass. These grippign members are preferably adjustable gripping members adapted to be controlled by the central control unit of the apparatus in accordance with said measure or measures of the carcass in question.

The above quality testing means preferably comprise a number of probe units, preferably 18 probe units, each comprising an insertion measuring probe, the probe units preferably being mounted on carriages the positions of which may be adjusted individually and controlled by the central control unit of the apparatus in accordance with said measure or measures, i.e. in accordance with the size of the carcass in question, and each carriage preferably comprises devices for a joint movement of the probe units associated with the carriage in any direction in one plane, as it is possible when the carriage is positioned correctly relative to the carcass in accordance with said measure or measures, by a single movement to insert the probe units associated with the carriage into the carcass instead of inserting the probe units into the carcass individually. In accordance with the preferred embodiment of the apparatus of the invention these devices comprise a first carriage system and a second carriage system mounted on said first carriage system, the carriage systems being adapted to move perpendicularly to each other driven by associated fluid drive mechanisms, i.e. hydraulic or pneumatic dirve mechanims. Said carriage systems may, of course, in an alternative embodiment, be driven ty electric motores, and the probes in such an electrical embodiment may be driven mutually independently, preferably controlled by the central control unit of the apparatus. As described above, the individual probe unit is preferably based on the light reflection detection principle and in accordance with the probe unit descirbed in the above Danish patent application they are formed as through-going piston rod arranged in a cylinder, and one end of the piston rod serves as an insertion probe and comprises means for measuring light reflection, and the other end of the piston is connected to a position detecting device for detecting the travel or position of the piston rod which makes it possible, by detection of the travel or position of the piston rod, to determine transitions between tissues, and in the case where it is impossible to insert the insertion probe further into the carcass owing to an impediment, for example a bone, it is possible in a simple manner to detect such a blockage as the piston rod does not perform any travel.

A particular aspect of the present invention relates to branding objects. More specifically, an apparatus is provided for branding objects especially carcasses of animals, which apparatus has a branding iron comprising a rod-shaped or tubular member and branding elements arranged at a first end thereof, means for supporting the object to be branded, means for heating the branding elements of the branding iron and means for providing a mutual displacement of the supporting means and the branding iron, and upon this mutual displacement, for bringing the branding elements of the branding iron into contact with the object to be branded.

It is generally known to mark objects such as carcasses by branding, i.e. by bringing a branding iron which has been heated to a high temperature, for example to a temperature of 500-1000°C, into contact with the object, whereby a durable marking of the object is provided. The technical literature, particularly the patent literature, describes numerous apparatuses for branding and further separate branding irons for providing such branding. Reference is made to Applicants' Danish patent application No. 5025/81 which patent application describes an assembly for branding carcasses, especially carcasses of hogs. This known assembly is characterised by a reliable marking of the object or the carcass to be branded, an efficient branding of the individual carcass being obtained with a high iron temperature even in the case where several assemblies in one and the same machine produce several brands at different positions of the carcass. It should be noted, however, that with this known assembly, only one and the same marking of objects, especially carcasses, may be obtained, unless the exchangeable branding blocks of the assembly are exchanged. In a high-speed marking system, especially a system in a slaughterhouse in which carcasses are forwarded in a transport system, such as a gliding conveyor, at a rate of 200-400 carcasses per hour, it is, however, not possible to provide an individual marking of the objects, especially the carcasses, by exchanging the branding blocks in this known assembly, as such a manual operation will partly be much too time-consuming, partly delay the branding process considerably, as newly inserted branding blocks have to be heated first to the above high branding iron temperature for providing an efficient marking.

It is further known to carry out marking of objects by inking and particularly to provide ink stamp aggregates by means of which an individual marking of the objects may be obtained, the ink stamp assembly having movable ink stamp parts which may be changed in accordance with a desired marking, for example for providing a consecutive numeration of objects, for providing a date stamp, etc. An ink stamp assembly of this type is described in published European patent application No. 0046704.

However, in slaughterhouses it is preferred to carry out marking by branding in order to exclude highly coloured and most often poisonous ink means from the slaughterhouses, as a branding of carcasses by means of ink stamps in many cases does not yield an efficient marking. It is, of cours, possible to provide a large number of individual branding irons in consecutive sections in an apparatus according to the invention for providing a partial classification stamping or branding on the individual sections of each carcass in accordance with this previous quality determination. Such a solution comprising several consecutive part classification stamps is, however, partly a very space consuming, partly a very expensive technical solution.

Consequently, there is a need for an apparatus for branding objects, which apparatus renders it possible to carry out individual branding of the individual object. A solution to this technical problem based on the art known from the ink stamps is not possible as certain parts of a branding iron is heated during use or operation to an extremely high temperature of, for example 500-600°C, which high temperature would directly destroy an iron intended for branding and formed in the same manner as the known ink stamps.

Consequently, in accordance with this particular aspect of the present invention an apparatus of the type mentioned above for branding objects is provided, which apparatus renders it possible to provide an individual branding of the objects, especially the carcasses, in principle in the same manner as with the known ink stamp assemblies having movable stamp parts for the provision of the individual marking of the objects.

The apparatus is characterized in that the branding elements of the branding iron comprise at least two sets of branding elements, each set consisting of one of several individual branding elements, that the branding iron further comprises positioning means arranged at the other end of the rod opposite said first end, for changing the branding iron between a number of positions corresponding to the number of sets of branding elements, in which positions the respective sets of branding elements are brought into contact with the object to be branded by the provision of said mutual displacement of the supporting means and the branding iron, and that the rod-shaped or tubular member of the branding iron has such a length and/or is cooled in such a manner that the transmission of heat from the branding elements heated by means of the heating means to the positioning means of the branding iron is restricted to such an extent that a thermal action harmful or destructive to the positioning means is avoided.

The recognition on which the invention is based, is, as will be understood, the recognition that by a physical separation of the positioning means and the heated branding elements, and by dimensioning the rod connecting the branding elements and the positioning means and/or by cooling the rod-shpaed or tubular member it becomes possible to restrict the transmission of heat from the heated branding elements to the positioning means and thus to avoid damage or even destruction of the positioning means which typically comprises electrical, hydraulic or pneumatic driving devices for providing the branding iron change between the individual positions. Such driving devices and any bearings, which may be roller bearings or gliding bearings having seat components which are lubricated by lubricants or are made from a wear-resistant plastic material, obviously cannont tolerate heating to a temperature of 500-100°C. The length of the rod-shaped or tubular member of the branding iron satisfying the characteristic feature of the invention that the transmission of heat from the heated branding elements to the positioning means of the branding iron is restricted obviously depends on the material from which the rod-shaped or tubular member of the branding iron is made. If the rod-shaped or tubular member of the branding iron is made from a material having a low heat transmission coefficient, especially a thermally insulating material, the length of the rod for satisfaction of the characteristic feature of the invention will, of course, be smaller than in the case where the rod-shaped or tubular member of the branding iron is made from a material having a high heat transmissin coefficient. However, in accordance with the invention, a material having good heat conductivity or a high heat transmission coefficient may be used, provided that the rod-shaped or tubular member of the branding iron is cooled. In this case thermal detecting means may be provided for protecting the positioning means, for monitoring the temperature of the rod-shaped or tubular member of the branding iron and controlling and interrupting the heating of the branding elements, if necessary, in case the cooling of the rod-shaped or tubular member of the branding iron turns out to be insufficient. The cooling of the rod-shaped or tubular member of the branding iron may, in accordance with well-known technical principles, be provided by forced air cooling or liquid cooling and possibly combined with heat exchange.

In accordance with an embodiment of the apparatus according to this aspect of the invention the sets of branding elements may be arranged in such a manner that a displacement of the branding iron in the longitudinal direction of the rod and/or a rotation of the branding iron around the axis of the rod changes the branding iron between said positions, the positioning means according to this embodiment being adapted to provide a linear displacement of the branding iron in the longitudinal direction of the rod and/or a rotation of the branding iron around the axis of the rod. Consequently, in accordance with this embodiment, the individual set of branding elements may described any combination of rotational movement and linear movement, thus especially a movement along a helical line, the sets of branding elements suitably being arranged helically along the axis of the rod. In case the positioning means are adapted to provide a linear displacement of the branding iron in the longitudinal direction of the rod, the sets of branding elements are preferably arranged consecutively and possibly on part of a curved surface, for example a spherical surface. In the general case, the positioning means are adapted to provide a combination of a linear displacement and a rotation of the branding iron, and the branding elements are also suitably arranged on, for example, a spherical surface.

In accordance with the presently preferred embodiment of the apparatus according to this aspect of the invention, the branding elements are arranged peripherally and at the same mutual peripheral distance around the rod-shaped or tubular member of the branding iron, the positioning means being adapted to provide a step-wise rotation of the branding iron around the axis of the rod corresponding to the peripheral axis. In this preferred embodiment of the apparatus according to the invention, consequently, the branding iron is only rotated around its axis of the rod is made, whereas no linear displacement of the branding iron is produced in the longitudinal direction of the rod.

In accordance with a further embodiment of the apparatus according to this aspect of the invention the branding iron comprises a total of six branding elements each having a peripheral extent corresponding to an angle of 60°, the positioning means having a ratchet device adapted to permit a rotation of the branding iron in one direction and to prevent rotation of the branding iron in the opposiet direction past said positions.

As already mentioned, the positioning means may be formed as electrical, hydraulic or pneumatic means for providing the change of the branding iron between said positions. In the preferred embodiment of the apparatus according to this aspect of the invention, in which embodiment the positioning means comprise a ratchet device, the positioning means advantageously further comprise a cylinder cooperating with this ratched device for providing the step-wise rotation of the branding iron. The cylinder may be a hydraulic or pneumatic cylinder, however, the cylinder is preferably a pneumatic cylinder.

The heating of the branding elements by means of the heating means may be made in any suitable manner, for example directly or indirectly. Thus, the heating means may be electric heating means which may be implemented in accordance with well-known principles such as the resistance heating principle, the induction heating principle or the high frequency heating principle. The heating may also be provided by mens of a suitable heat transmission fluid which is heated to the desired temperature and is circulated in a closed circulation circuit between a heat source and the branding elements. In this case the heat source may also be an electric heat source or a heat source in which a fossil fuel such as oil or gas is burned. In the presently preferred embodiment of the apparatus according to this aspect of the invention, the branding elements are heated directly by means of a gas burner, and in this embodiment the heating means consequently comprise gas burner means, flame monitoring means and spark ignition or preferably glow ignition means, the combustion of the gas being monitored in a manner known per se by means of the flame monitoring means, and the gas being ingnited or re-ignited, if necessary, if the combustion has been interrupted intentionally or unintentionally, by means of the ignition means.

To restrict the heat radiation from the branding elements and thus the energy consumed for heating the branding elements it is preferred that the branding elements of the branding iron are at least partially enclosed in a heat insulating shield. This shield consequently serves to restrict the heat radiation from the heated branding elements. The branding elements may be movable relative to the shield, the set of branding elements which is brought into contact with the object to be branded by provision of the mutual displacement of the supporting means and the branding iron, being passed out through this opening. In this case the shield may, in a simple manner, be arranged movably relative to the branding iron, possibly be movable and spring-biassed, the shield being retracted by provision of said mutual displacement of the supporting means and the branding iron to uncover the relevant set of branding elements which are brought into contact with the object to be branded. Alternatively, the shield may be formed in such a manner that this set of branding elements, which are brought into contact with the object to be branded by provision of the mutual displacement of the supporting means and the branding iron, extends through the opening in the shield.

In connection with branding of carcasses of animals it has turned out that it may be desirable to stretch or tighten the area of the carcass on which area a branding is to be produced for providing an easily legible branding. Consequently, the apparatus according to this aspect of the invention preferably has a spring-biassed abutment means for abutment against the surface of the object to be branded, immediately before a set of branding elements is brought into contact with this object by said mutual displacement of the supporting means and the branding iron.

It is further particularly preferred that the mutual displacement between the supporting means and the branding iron is provided by the branding iron being moved towards the supporting means in which the object, especially the carcass to be branded, is supported or kept fixed. Especially, the branding iron may be forwarded towards the object supported by or fixed in the supporting means, especially the carcass fixed in the supporting means, by a swinging movement.

In accordance with this aspect of the present invention a branding iron for use in an apparatus according to this aspect of the invention and with any of the characteristics of the branding iron of the above described apparatus is also provided.

The invention will now be explained in greater detail with reference to the drawing of which

Fig. 1 is a perspective view of an apparatus according to the invention for treatment and examination of carcasses of hogs and especially for classification and marking of carcasses, in which apparatus carcasses of hogs are consecutively and suspended in a frame passed through a number of stations in the apparatus,

Fig. 2 is a schematical and perspective view of a length determination device shown in Fig. 1,

Fig. 3 is a schematical and perspective view of a device also shown in Fig. 1 for height adjustment of the head holding parts of said frames,

Fig. 4 is a schematical and perspective view of a device for determining the foreleg position,

Fig. 5 is a schematical and perspective view of a device for determining the collarbone position,

Fig. 6 is a schematical and perspective view of an insertion sampling probe unit,

Fig. 7 is a schematical and perspective view of a detail of the frame advancing mechanism shown in Fig. 1,

Figs 8 and 9 are schematical views illustrating details of a part of the transport system of the apparatus shown in Fig. 1 for transportation of the frames,

Fig. 10 is a schematical and perspective view of a part of a marking station in the apparatus shown in Fig. 1,

Fig. 11 is a schematical and perspective view of a first type of branding iron in the marking station shown in Fig. 10,

Fig. 12 is a schematical and perspective view of a second type of branding iron in the marking station shown in Fig. 10,

Figs. 13 and 14 are perspective and - schematical view of details of the types of branding irons shown in Figs. 11 and 12,

Fig. 15 is a schematical and perspective view of a branding apparatus according to the invention, and

Figs. 16 anc 17 are perspective and - schematical view of details of the branding apparatus shown in Fig. 15.

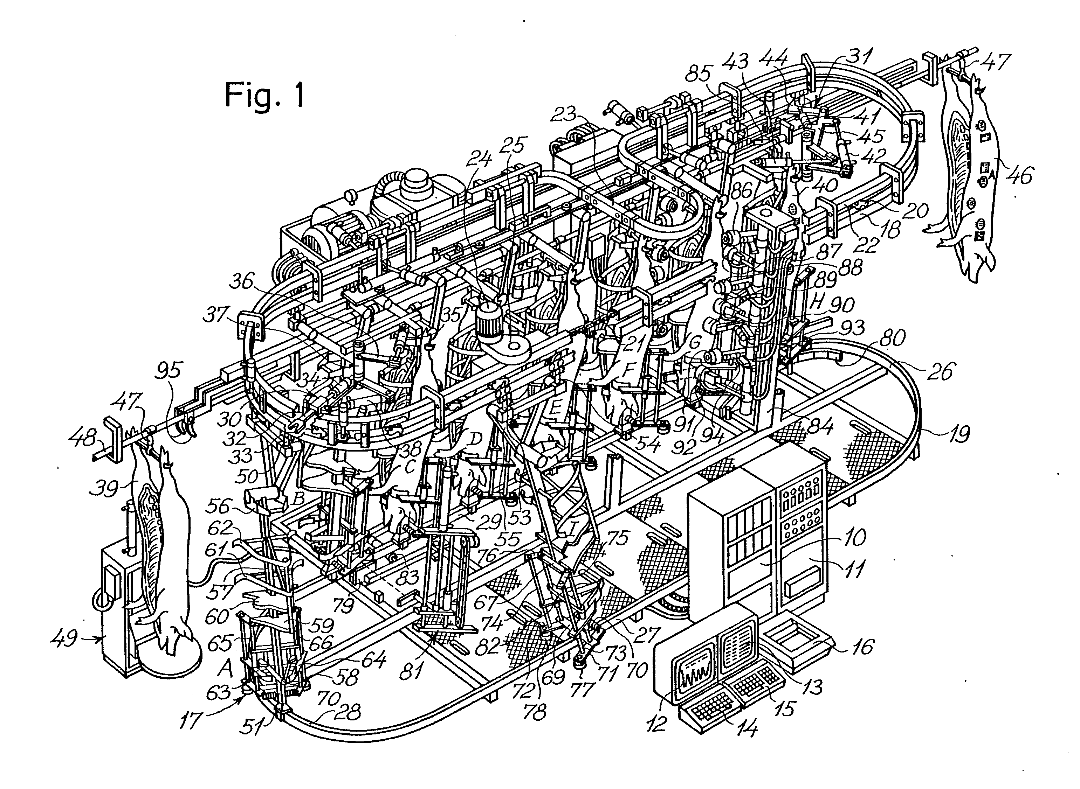

Fig. 1 shows an apparatus or part of an apparatus according to the invention and for carrying out a method according to the invention of treatment or examination of carcasses of hogs which are passed through the part of the apparatus shown in Fig. 1. The apparatus is a computer controlled apparatus, whose computer is shown schematically in Fig. 1 comprising two cabinets 10 and 11 containing one or more CPUs (central processing units), various memories, etc. and two terminals 12 and 13 with associated keyboards 14 and 15, respectively, and a printer 16. The computer receives information from various detectors in the apparatus and controls the operation of the apparatus by means of fluid pressure cylinders, i.e. hydraulic and pneumatic driving devices, other driving devices, for example electric motors, being possible for use in connection with an alternative embodiment of the apparatus according to the invention.

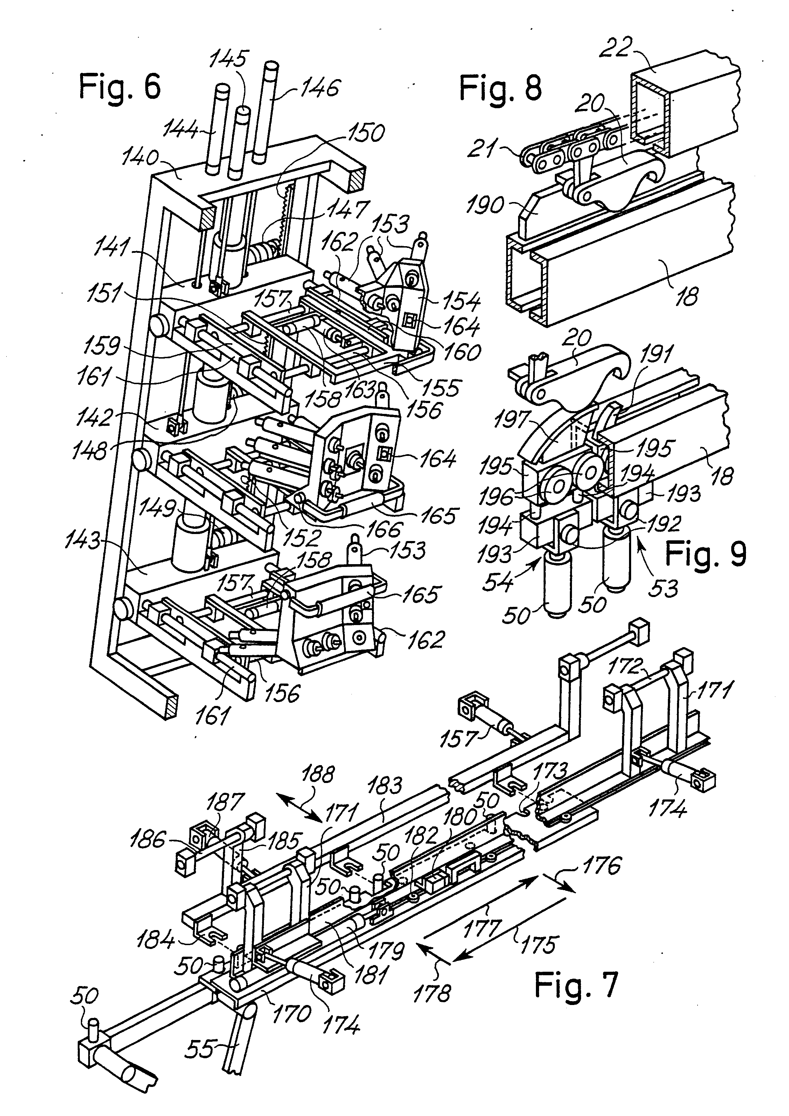

The apparatus comprises a transport system with transport and fixation frames 17. In the embodiment shown in Fig. 1, the apparatus comprises nine fixation frames, however, the number of fixation frames is arbitrary and may be freely chosen, as part from one frame per station in the apparatus, only one or a few frames must be available for introduction or reception of carcasses of hogs which are introduced into the apparatus. The frames 17 are guided in a closed path by means of an upper and a lower rail 18 and 19, respectively, defining the same endless path. The rail 19 serves to support and guide the individual frame as will be explained in greater detail below with reference to a description of a single frame, and the upper rail 18 serves to hold and suspend the frames 17 and further cooperates with carriers 20 which, as will be explained in detail below with reference to Figs. 8 and 9, are driven by a chain 21 which is enclosed in a chain housing 22 mounted above the rail 18, which chain housing 22 substantially follows the same closed endless path as the rail 18 except for a length 23 on which the chain housing 22 forms a loop serving the purpose of rendering possible a tightening of the chain 21 enclosed in the chain housing 22. The chain 21 is driven by an electric motor 24 with an associated gear with a constant, but adjustable clockwise velocity. The path along which the frames are forwarded, is defined, as mentioned above, by the rails 18 and 19, and this path consists of a semicircular path section corresponding to a semicircular rail section 26 of the rail 19, a linear path section corresponding to a linear rail section 27 of the rail 19, and furthermore of path sections corresponding to another semicircular rail section 28 and another linear rail section 29 of the rail 19. Said clockwise movement is consequently a movement corresponding to a movement from the rail section 26 to the rail section 27 and further to the rail section 28 and to the rail section 29, etc., in the endless path defined by the rails 18 and 19. The chain 21 mentioned above passes the individual frame from a position corresponding to approximately the middle of the semicircular rail 26 to a position approximately corresponding to the middle of the semicircular rail section 28 at a substantially constant rate by means of one of the above carriers 20.

In the part of the closed path corresponding to the linear rail section 29, the frames 17 are intermittently passed along with a single carcass of a hog fixed on each frame through a number of individual treatment or examination stations, the intermittent movement of the frames along the linear path corresponding to the linear rail section 29 being provided not by means of the continuously working chain conveyor, but by means of an intermittently working forwarding mechanism, as will be explained in greater detail below with reference to Fig. 7.

For transfer of a frame from the continuous movement described above and to the intermittent forwarding or movement through the treatment or examination stations and for transfer of the frames from this intermittent movement to the continuous movement, the apparatus comprises a frame introduction swivel arm assembly 30 and a frame ejection swivel arm assembly 31, respectively. When the frame 17, which is arranged in a position corresponding to the middle of the semicircular rail section 28, which position is indicated by the reference A, is to be forwarded to a position B, the frame introduction swivel arm assembly 30 is actuated. The frame introduction swivel arm assembly 30 has an axially outwardly displaceable part 32 having a claw-shaped part 33 which, by axial outward displacement, is brought into engagement with a pin 50 of the frame 17 by actuation of a fluid pressure cylinder 34 which is preferably a pneumatic cylinder. After the engagement of the claw-shaped part 33 with the pin 50, another fluid pressure cylinder 35, which is also preferably a pneumatic cylinder, is acutated, and the cylinder 35 causes the frame introduction swivel arm assembly 30 to turn from a position corresponding to the position A towards a position corresponding to the position B by means of an arm 36 which is fastened to the assembly 30 via a bushing 37, and at the same time a third fluid cylinder 38 which is also preferably a pneumatic cylinder is actuated to displace the axially displaceable part 32 a further distance in the axial direction of the swivel arm assembly.

The frame arranged in position B is, as is evident from Fig. 1, ready to receive a carcass of a hog which is introduced into the cyclically working apparatus from a continuously working apparatus or system existing in a slaughterhouse. Such as carcass is shown at the left-hand side of Fig. 1 and is designated 39. After reception of the carcass 39 in position B, the frame is forwarded with the carcass 39 received and fixed therein from position B to a position C and further via positions D, E, F and G corresponding to individual treatment and/or examination stations to a position H in which the carcass is removed from the frame, whereupon the now processed and examined carcass, designated 40 in Fig. 1, is further forwarded by the existing, continuously working transport system mentioned above. The frame is removed from the carcass by means of the frame ejector swivel arm assembly 31 mentioned above. Like the frame introduction swivel arm assembly 30 described above, this frame ejector swivel arm assembly 31 is an assembly which is actuated by a total of three fluid pressure cylinders 41, 42 and 43 and has an outer articulated arm consisting of an outer claw-shaped part 44 and an inner swivel arm part 45 to which the claw-shaped part 44 is swingably or pivotably fastened. The ejection takes palce by the claw-shaped part 44 being first brought into engagement with the pin 50 of the associated frame by actuation of the fluid pressure cylinder 41, whereby the claw-shaped part 44 is made to swing clock-

wise relative to the swivel arm 45. whereupon the fluid pressure cylinder 42 is actuated to swing the arms 44 and 45, whereby the frame is passed round in the part of its path corresponding to the semicircular rail section 26, and at the same time the third fluid pressure cylinder 43 is actuated. After this last ejection step the frame has been moved to a position corresponding to the middle of the semicircular rail section 26, from which position the frame is forwarded to a position designated I in Fig. 1, and further to position A by the continuously working chain conveyor described above. The introduction and the frame ejection are controlled by the central computer described above, which receives information regarding the state and position of the frames from position detectors in the form of capacitive or, preferably, inductive sensors which monitor the movement of the frames along the closed path defined by the rails 18 and 19.

After ejection of the frame from the associated carcass the latter is further passed along in the continuously working transport system already existing in the slaughterhouse and mentioned above. Such a carcass is shown in the upper right-hand corner of Fig. 1 and is designated 46, which carcass 46 is transported away from the apparatus shown in Fig. 1 by means of the continuously working transport system.

During transportation in the already mentioned existing and continuously working transport system in the slaughterhouse and during the movement through the apparatus shown in Fig. 1 the carcasses are permanently mounted on gambrels shown in Fig. 1 and designated 47. The gambrels 47 are in their turn suspended in a grip round a rail 48 extending through the apparatus shown in Fig. 1 and further constituting part of or constituting an extension of a guide rail in the continuously working transport system from which the carcasses are transferred to the apparatus shown in Fig. 1, and to which the carcasses are transferred after the completion of the treatment and examination, etc., in the apparatus shown in Fig. 1. It should be noted, however, that the gambrels do not engage with or hang on the rail 48 while the associated carcasses are passed through the intermittently working apparatus shown in Fig. 1.

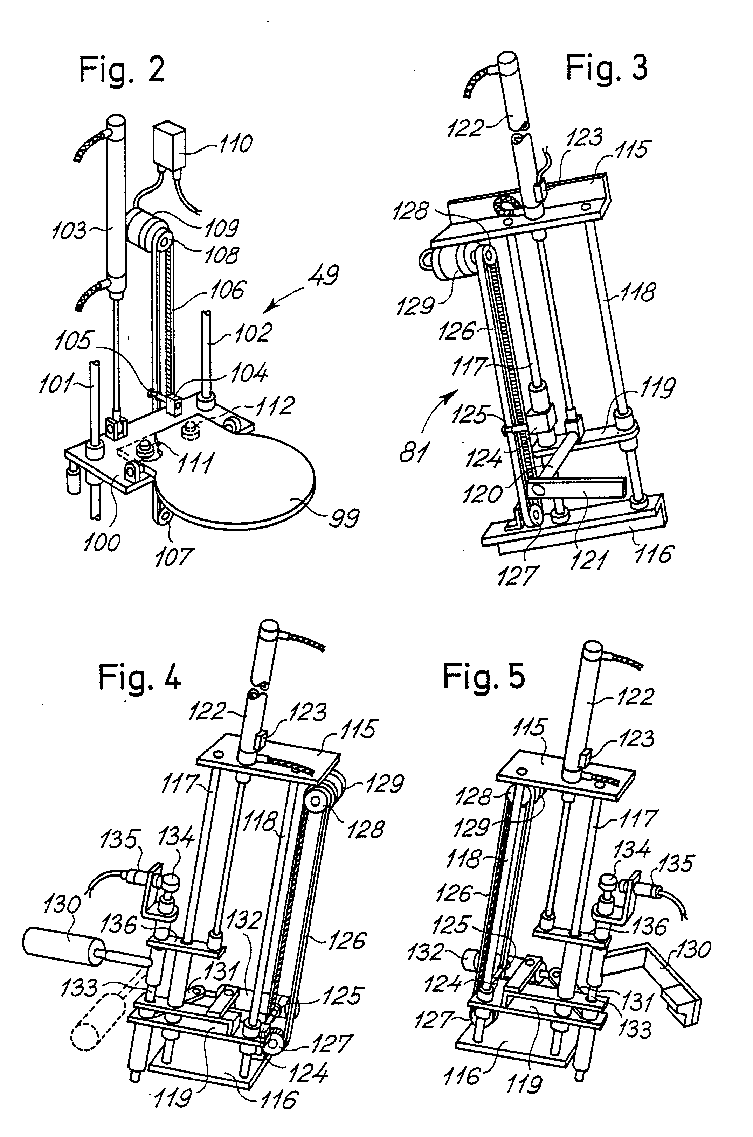

In a first station or a pre-apparatus station, seen relative to the intermittently working apparatus with the frames 17, in which station a length measuring device 49 which will be described in greater detail below with reference to Fig. 2 is arranged, a detrmination of the position of the snout of the carcass in question is made and thus a determination of the total length of said carcass, as the position of the snout of the carcass, as a result of the suspension of the carcass in the gambrels 47 which are in their turn suspended in the rail 48, unambiguously determines the length of the carcass.

Now, the structure of a single frame 17 will be described in greater detail. The frame 17 has a lower rail guide part 51 which is guided on the lower rail 19, and two upper guide rail parts 53 and 54 of which the rail guide part 54 comprises the above-mentioned pin 50. The parts 53 and 54 are mounted on a triangular frame 55 the bottom tip of which is fastened to a support 56 from which a substantially vertical beam 57 extends to the rail guide part 51. On the beam 57 five carcass supports 58, 59, 60, 61 and 62 are mounted spaced apart, which carcass supports are adapted to support a carcass split along its longitudinal direction, for example the carcass 39 shown at the left-hand side of Fig. 1 and being introduced into and arranged on the frame shown in Fig. 1 in position B. Each frame 17 further comprises two gripping arms 63 and 64 which are mounted in bushings on rods 65 and 66, respectively, which are parallel to the beam 57 and fastened thereto via rods 67 and 69. The gripping arms 63 and 64 are vertically displaceable, said bushings of the gripping arms being displaceable on the rods 65 and 66. The gripping arms 63 and 64 further constitute a swivel arm comprising lower arms 71 and 72, respectively, which are parallel to the gripping arms 63 and 64, respectively, and which are biassed by a spring 70 towards a normally closed position of the gripping arms, two vertical rods 73 and 74, respectively, which are rigidly connected to the associated lower arms 71 and 72, respectively, and extend through openings in the associated gripping arms 63 and 64, respectively, to permit said vertical displacement of the gripping arms, and two upper arms 75 and 76, respectively, rigidly connected to the rods 73 and 74, respectively. Each of the swivel arm constructions 63, 71, 73 and 75, and 64, 72, 74 and 76, respectively, constitute rigid constructions which permit transfer of forces on the lower arms 71 and 72, respectively, to the associated gripping arms 63 and 64, respectively. As mentioned above the spring 70 biasses the lower arms 71 and 72 and thus the associated gripping arms 63 and 64, respectively, towards a closed position shown in positions F and A in Fig. 1.

The opening of the frames to enable the carcasses to be introduced into the frame and to be ejected therefrom the lower arms 71 and 72 have rollers 77 and 78, respectively, adapted to engage with roller guides when the associated frame is forwarded from position A to position B, and when the frame is forwarded from position G to position H. In Fig. 1 two such roller guides are designated 79 and 80, but it is understood that apart from these roller guides serving to engage with the roller 78 roller guides ae provided on the opposite side of the rail 19 to engage with the roller 77. When the frame is forwarded from position A to position B or from position G to position H, the rollers 77 and 78 engage with the corresponding roller guides whereby the rollers are forced against each other against the action of the spring 70, whereby the swivel arms of the swivel arem construction described above are passed to an open position giving access to the introduction or the ejection of a carcass. As is evident from the positions C, D, E, F, and G, shown in Fig. 1, the gripping arms 63 and 64 grip around the neck of a carcass arranged on the associated frame and thus ensure safe retention of the carcass in the question on the associated frame. For positioning the gripping arms 63 and 64 which are interconnected via a gripping arm connecting rod 82, the apparatus comprises a gripping arm positioning device 81 which is a height positioning device shown in Fig. 1 in an extended position and which will be explained in greater detail below with reference to Fig. 3. The gripping arm positioning device 81 provides a height positioning of the gripping arms 63 and 64 controlled by the computer of the apparatus on the basis of the height determination made in the device 49, as the computer determines, in accordance with an empirically determined linear algorithm, the position of the neck of the carcass on the basis of the carcass in question. In accordance with this determination of the position of the neck, the gripping arms are positioned correctly in alignment with the neck of the carcass in question by means of the gripping arm positioning device 81. It should be noted that the opening of the frames in positions B and H, respectively, may take place in several manners. Thus the opening at position H takes place by the rollers 77 and 78 being brought into engagement with two non-parallel guide rails of which one is shown in Fig. 1 and is constituted by the above mentioned roller guide 80 which defines a narrowing in position H, in which position the rollers 77 and 78 are guided towards each other. In position B the opening of the frame 17 is made in another way, as the above mentioned roller guide 79 is connected with a corresponding roller guide arranged on the opposite side of the rail 19 via a fluid pressure cylinder 83 which is controlled by the computer of the apparatus to guide the roller guide 79 and the associated roller guide arranged on the opposite side of the rail 19 towards each other and thus guide the rollers 77 and 78 towards each other for opening of the frame arranged in position B.

The apparatus shown in Fig. 1 is operated and controlled by the computer of the apparatus in the following manner. The carcass 39 is introduced in the transport system by means of a telescopically displaceable claw 95 which passes the carcass into the frame which is arranged in position B and is opened. Simultaneously with the introduction of the carcass, the gripping arm positioning device 81 positions the gripping arms 63n and 64 in alignment with the neck of the carcass controlled by the computer on the basis of the measurement of the length of the carcass in question determined in the length measuring device 49. After closure of the frame by actuation of the cylinder 83 the frame is transferred with the carcass fixed in a safe and reliable manner by means of the gripping arms 63 and 64 to position C in which a determination of the position of the foreleg and collarbone of the carcass in question is made by means of devices which are not shown in Fig. 1, but will be described in greater detail below with reference to Figs. 4 and 5. The foreleg and collarbone position determination in position C and the measurement of the total length made by the device 49 are processed in the computer on the basis of empirical data. From position C the frame is transferred with its associated carcass to position D in which, by means of measuring means (not shown) a sample may be taken, for example with a view to determining any sexually determined smell for male hogs or boars, the so-called boar smell. In position E to which the frame with associated carcass is passed from position D, a quality determination is made by means of insertion probes which are not shown in Fig. 1, but will be described in greater detail below with reference to Fig. 6. The positioning of the probes is controlled by the computer of the apparatus on the basis of the determination of the total length of the carcass in question and on the basis of the determination of the position of the foreleg and the collarbone. In position F an alternative quality determination or examination may be made by means of means not shown, and in position G a branding is made of the carcass on the associated frame arranged in position G by means of positionable stamps which are mounted on a stamp support 84. By transfer from position G to position H the frame is opened, and the frame is removed from the carcass by means of the frame ejection swivel arm assembly 31 described above, whereupon the carcass 40 and the gambrel 47 which are held during the frame ejection by a stop device, are pushed out of the intermittently working apparatus by means of a carrier 85 driven by a fluid pressure cylinder, and are transferred to the continuously working transport system in the slaughterhouse. This transport system forwards the carcass 46 to further treatment, cutting up, sawing up, etc. This transport system and any cutting up and carving means arranged at this continuously working transport system may possibly be controlled by or cooperate with the computer of the intermittently working apparatus shown in Fig. 1 and thus receive information on the measuring, quality determination, classification and branding carried out in the apparatus shown in Fig. 1.

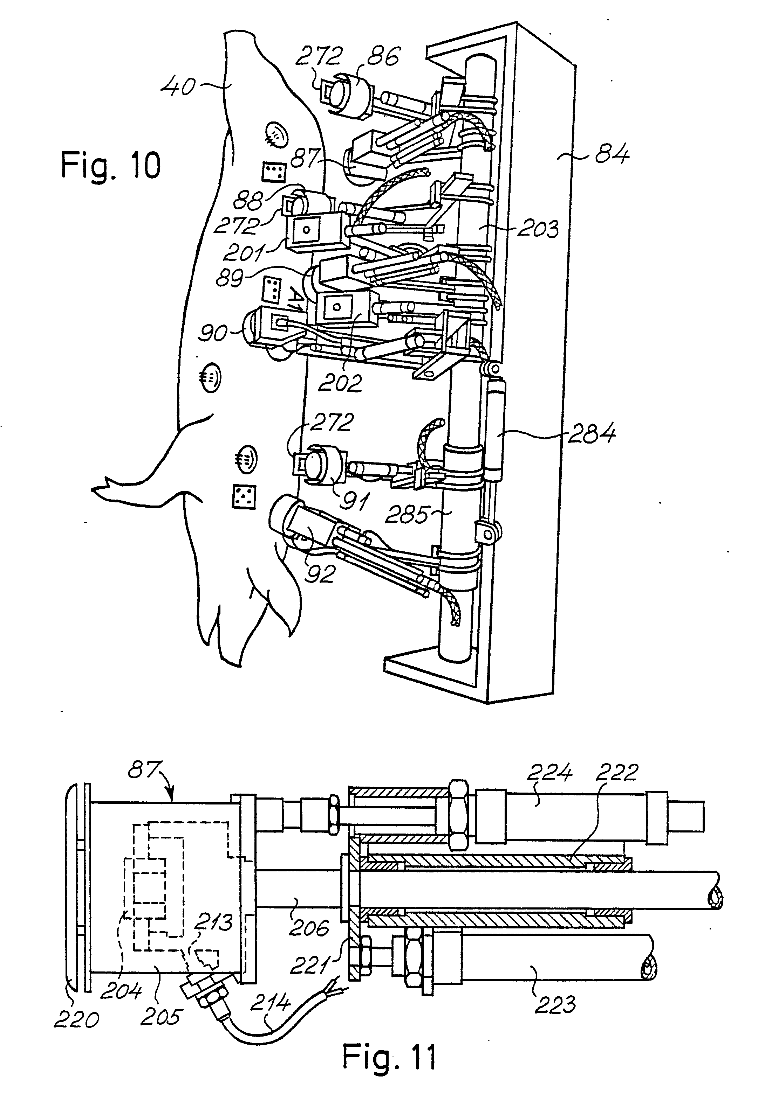

The marking or branding in position G is made by means of a total of eighteen branding assemblies to be described in greater detail below with reference to figs. 10-17, of which assemblies nine are arranged one one side of the carcass, and of which the other nine are arranged on the other side of the carcass. These branding assemblies are driven and actuated in pairs. Five upper branding assemblies 86, 87, 88, 89, and 90 of each of these nine branding assemblies are preferably stationary while two lower branding assembliew 91 and 92 of each of these seven branding assemblies are preferably positionable. Two of the total of nine branding assemblies are not shown in Fig. 1. The two positionable branding assemblies 91 and 92 are driven by fluid pressure cylinders 93 and 94, respectively. It should be noted that it may be necessary for branding of carcasses of extremely varying lengths to provide more than two of the branding assemblies as positionable branding assemblies which are controlled by the computer of the apparatus on the basis of empirical data for the type of slaughter animal in question in accordance with the length determination made in the device 49 and the foreleg and collarbone position determinations made in position C. Thus, in connection with hogs it has proved that the conversion of the determinations made in the device 49 and in position C of said three outer dimensions, the total length, the height or position of the foreleg and the height or position of the collarbone, into control data for positioning of the insertion probes in position E and for control of the positionable assemblies 91 and 92 may be made in accordance with a linear algorithm, as carcasses of hogs in practice substantially satisfy such a linear algorithmic relation. In a few cases it may be necessary to carry out a repositioning of, for example, an insertion prob as it has hit a massive formation, for example a rib, by moving such an insertion probe a distance up or down corresponding to for example one half rib distance, which in practice corresponds to about 1-2 cm, in the preferred embodiment 16 mm.

Apart from the above described components, the apparatus shown in Fig. 1 comprises various means associated with the pressure fluid actuated cylinders such as air compressors, hydraulic pumps, compressed air containers, compressed air ducts, hydraulic pipes, etc., which components have been shown in Fig. 1, but have not been described in greater detail as they constitute components known per se.

Fig. 2 shows in greater detail the length measuring device 49 also shown in Fig. 1. The device 49 comprises a plate 99 which is mounted tiltably on a supporting plate 100 which may be reciprocated in bushings on vertical rods 101 and 102 by means of a fluid pressure cylinder. A support 104 for a toothed belt driving roller 105 is further mounted on the plate 100 which toothed belt driving roller engages with a toothed belt 106 mounted on a lower toothed belt roller 107 and an upper toothed belt roller 108 which is in its turn mounted on the shaft of a pulse generator 109 which generates pulses corresponding to the rotation of the roller 108, which pulses are transferred via an amplifier/converter built into a housing 110 to the computer of the apparatus shown in Fig. 1. The pulse generator 109 may be a tacho generator, a capacitive or inductive generator comprising a rotary armature or preferably an electric motor which is driven by a pulse generator. The device 49 further comprises switches 111 and 112 which detect whether the plate 99 tiltably mounted on the plate 100 is in the position shown in Fig. 2 or whether the plate 99 is tilted as a consequence of the upper side of the plate 99 having met an impediment at the upward movement of the plate 100, especially having contacted the snout of a hog as shown in the left-hand side of Fig. 1. In the apparatus shown in Fig. 1, the height of the snout is detected and thus the length of the carcass is preferably detected simultaneously with or immediately before the carcass 39 is forwarded into the intermittently working apparatus by means of the claw 95.

The device 49 operates in the following manner. The plate 99 is moved upwards from a bottom position at a substantailly constant rate, the pressure cylinder 103 elevating the plate 100 and thus the plate 99 tiltably mounted on this plate. When the plate 99 meets the lower point of a carcass suspended over the plate 99, the plate 99 tilts, whereby the switches 111 and 112 are activated. During this movement the pulse generator 109 has generated a number of pulses corresponding to the current height relative to the bottom position preferably determined by a sensor in a manner which will be described below with reference to Figs. 3, 4, and 5, and the activation of the switches 111 and 112 together with the number of pulses generated during the vertical movement of the plate 99 and the plate 100 contain information about the height at which the plate 99 meets the snout of the carcass suspended over the plate 99 and thus, as the point of suspension of the carcass in question from the gambrel 47 in zthe rail 48, vide Fig. 1, is fixed relative to the support of thedevice 49, further contain information about the length of the carcass in question from hind legs to tip of snout. The calculation of this length is made in the computer on the basis of the information transferred from the switches 111 and 112 and the number of pulses generated by the pulse generator 109. On the basis of this information and these pulses and further on the basis of empirical data, as explained above, the computer determines the position of the neck of the carcass just measured and controls the gripping arm positioning device 81 shown in Fig. 1 and also in Fig. 3 accordingly.

Like the deivces described above with reference to Figs. 4 and 5 for determination of the position of the foreleg and the collarbone, the gripping arm positioning device 81 shown in Fig. 3 is based on the same detecting principle as the height measuring device shown in Fig. 2, i.e. the determination of a position based on the counting of distance pulses generated which distance is travelled. The device 81 comprises two stationary supports 115 and 116. Between the supports 115 and 116 two rods 117 and 118 extend and are fixedly connected thereto. On the rods 117 and 118 a cross beam 119 is displaceably mounted, and a crank lever construction comprising two arms 120 and 121 extends from this beam. The arm 121 is adapted to engage with the bottom side of the above described gripping arm 64, shown in Fig. 1, on the frame arranged in position B, and to move this gripping arm upwards. This upward movement is produced by a fluid pressure cylinder 122 the piston of which is connected with the beam 119. The pressure cylinder 122 differs from the pressure cylinder shown in Fig. 2 in that it has an end stop detector 123 for detecting a bottom position of the cylinder piston and thus of the beam 119, the arms 120 and 121 and the gripping arms 63 and 64. This detector 123 may be of any type, for example a so-called proximity detector and may be based on a capacitive or preferably an induction detection principle.

The device 81 further comprises a support 124 mounted on the beam 119, a toothed belt driving roller 125 mounted on the support 124, a toothed belt 126, two toothed belt rollers 127 and 128 and an pulse generator connected to the toothed belt roller 128 and drivent thereby. The components 124-129 correspond to the above described components 104-109 shown in Fig. 2.

The gripping arm posiitoning device 81 is operated in the following manner. The fluid pressure cylinder 122 moves the beam to a bottom position controlled by the computer of the apparatus, and this bottom position is detected by means of the detector 123. On the basis of the length determination made by means of the device 49, the fluid pressure cylinder 122 is actuated controlled by thr computer to elevate the beam 119, the arms 120 and 121, and the gripping arms 63 and 64 which at some time are brought along by the airm 121 in the upward movement. The pulse generator 129 continues during this upward movement to gen-

14

erate pulses corresponding to the position determined by the computer regarding the gripping arm 63 and 64. and when the number of pulses generated by the pulse generator 129 coresponds to the position determined by the computer regarding the griping arms 63 and 64, the supply of pressure fluid to the fluid pressure cylinder 122 is discontinued or interrupted.

In Figs. 4 and 5, devices are shown for determining the height or position of the foreleg and the collarbone, respectively, of a carcass arranged in position C. As already mentioned these position detection or position determination devices are based on the height position determination or travel distance determination principle described above with reference to Fig. 2. The devices shown in Figs. 4 and 5 are further of substantially the same construction as the construction shown in Fig. 3, for which reason identical reference numerals have been used for identical components. Instead of the arms 120 and 121 shown in Fig. 3 the devices shown in Figs. 4 and 5 comprise a detector arm 130 pivotably mounted on the beam 119. In a manner which will be explained below, the detector arm 130 is mounted on a rod 133 which is rigidly connected to an arm 131 which is in its turn connected with the piston of a fluid pressure cylinder 132. By actuation of the cylinder 132 the detector arm 130 shown in Fig. 4 may be swung from a position shown in full lines to a position shown in dotted lines. In the position shown in full lines the detector arm 130 is swung away from the path of movement of the carcasses so that they may freely be passed to and from the position C in the apparatus shown in Fig. 1, while in the position shown in dotted lines in Fig. 4, the detector arm projects into the path of movement of the carcasses, and when the detector arm is moved upwards as will be explained below, it may hit against a foreleg of a carcass being in the position C shown in Fig. 1.

Correspondingly, the detector arm 130 shown in Fig. 5 may be swung away from the path of movement of the carcasses and into the same and likewise, as will be explained below, may be moved upwards until the detector arm shown in Fig. 5 hits against the collarbone of a carcass. The detector arm 130 is mounted on the rod 133 in such a manner that the detector arm 130 may be reciprocated relative to the rod 133, the detector arm 130 being biassed by a spring towards an upper end position in which a body 134 connected to the detector arm 130 is arranged adjacent to a capacitive or inductive sensor 135. This upper end position for the detector arm 130 is determined by a beam 136 parallel to the beam 119, to which beam 136 the piston of the fluid pressure cylinder 122 is fastened.

The devices shown in Figs. 4 and 5 are operated in the following manner. When a carcass is in the position C shown in Fig. 1, the detector arms 130 shown in Figs. 4 and 5 are swung from positions shown in full lines in Figs. 4 and 5 are swung from positions not prevent or block the movement of the carcasses through the apparatus shown in Fig. 1, i.e. prevent or block the transportation of a carcass from and to the position C shown in Fig. 1, to positions shown in dotted lines in Figs. 4 and 5 in which the detector arms shown in Figs. 4 and 5 project into the path of movement of the carcasses. From a lower position detected by means of the detector 123 the detector arms 130 are moved upwards by the cylinders 122, and when the detector arm 130 shown in Fig. 4 hits the foreleg of a carcass, the detector arm 130 is moved downwards against the action of the above mentioned spring not shown on the drawings, whereby the body 134 is removed from the proximity of the detector 135, which results in the generation of a detection signal by the detector 135. Correspondingly, the detector 135 shown in Fig. 5 generates a detection signal when the detector arm 130 shown in Fig. 5 hits the collarbone of a carcass arranged in position C in Fig. 1. During the movement from the lower position or the bottom position and upwards the pulse generators 129 generate pulses which are converted by the computer of the apparatus into a distance travelled by the detector arms 130 and thus into an immediate height above the bottom position, and when the detectors 135 generate detection signals, the number of previously generated and detected pulses corresponds to the height or position of the foreleg (Fig. 4) and of the collarbone (Fig. 5).

Common to all the devices described above with reference to Figs. 2-5 is that the movement from the bottom position upwards is stopped or blocked when the intended function has been carried out, whereupon the devices are returned to their bottom positions and are thus ready to carry out a measurement, positioning or height determination during a next step of the intermittent operation of the apparatus.

Fig. 6 shows a test probe unit or a probe measuring station adapted to insert a number of probes, preferably a total of 18 probes into predetermined locations of a carcass which is at the position E shown in Fig. 1, aligned with the probe unit. The probe unit has a frame 140, three carriages 141, 142 and 143 which are vertically displaceable relative to the frame and individually positionable by means of fluid pressure cylinders 144, 145 and 146 and position detection devices corresponding to the pulse generators 109 and 129 described above, which pulse generators of the carriages 141, 142 and 143 are designated 147, 148 and 149, respectively, and are driven by racks 150, 151 and 152, respectively, contrary to the pulse generators described above which are all driven by toothed belts. The control of the fluid pressure cylinders 144, 145 and 146 and the position detection by means of pulse generators 147, 148 and 149 takes place in a manner completely analogous to the measurement, positioning and height determination described above. The structure of the carriages 141, 142 and 143 is completely identical, for which reason only the structure of one carriage 141 will be described. The upper carriage 141 supports a total of five insertion measuring probes which are preferably of the type described in Danish patent application No. 4247/86, and of which Fig. 6 shows a total of three probes which are designated 153. The measuring probes 153 are mounted in a supporting part 154 which defines a surface anatomically adapted to a carcass arranged in alignment with and adjacent to the unit or station, so that when the measuring probes 153 are correctly positioned controlled by the computer on the basis of the dimensions with regard to length, foreleg position and collarbone position determined for the carcass in question, they may be inserted correctly into intended locations on the carcass. For correct positioning of the measuring probs, the probes are mounted on the carriage 141 on a double carraige system, i.e. a system comprising two carriages which render possible movement in two directions at right angles to each other, and at a right angle to the direction of movement of the carriage 141 relative to the frame 140. The combination of the vertical movement of the carriage 141 in the frame 140 and this double carriage system consequently defines a three-dimensional, orthogonal system of movement for the measuring probes 153. The measuring probe support 154 is mounted on a chassis part 155 which is in its turn mounted displaceably on rods 156 and 157. The displacement of the chassis part 155 relative to the rods 156 and 157 in their turn constitute components of a chassis part further comprising two beams 159 and 160. The chassis part comprising the rods 156 and 157 and the beams 159 and 160 is in its turn axially displaceably mounted as a unit on rods 161 and 162, the axial displacment of which chassis part relative to the rods 161 and 162 is produced by means of a fluid pressure cylinder 163. While the positioning of the carriages 141, 142 and 143 is carried out in a predetermined manner, i.e. in an manner determined by the previously determined dimensions of the carcass in question, the measuring probes 153 are positioned by means of the above described double carriage system relative to the carcass by means of position detectors 164, a sensor arm 165 and a switch 166 cooperating therewith. The probes are positioned relative to a carcass arranged in alignment with and adjacent to the measuring probe unit by the fluid drive cylinder 163 first moving the latter chassis part or carriage and the first chassis part or carriage mounted thereon towards the carcass in question. When, during this movement, the sensor arm 165 meets the carcass, the arm 165 is bent, whereby the switch 166 is activated, and the activation of the switch 166 is detected in the computer of the apparatus, which computer interrupts the supply of pressure fluid to the fluid pressure cylinder 163. Then the computer of the apparatus activates the fluid pressure cylinder 158 which displaces the first chassis part or carriage in a direction perpendicular to the direction of the movement just described, the position detector 164 being placed in such a manner that a detection of an edge of a carcass by means of this position detection corresponds exactly to the intended or correct positioning of this first chassis part or carriage and thus of the whole carriage in question relative to the carcass. The support 154 and the corresponding supports of the carriages 142 and 143 and the positioning of the individual measuring probes of these supports are adapted to the physical arrangement of the carcass on the transport and fixation frame and further to the anatomy of the carcass. The middle carriage 142 further preferably has a detector which cooperates with the sensor arm on this carriage for further monitoring the insertion of the probes of the carriage 142 and for eliminating any risk that the rind of a carcass is scratched by the probe tips.

As described above, the frames in the transport system shown in fig. 1 move partly in a continuous movement without carcasses, partly in an intermittent movement with carcasses, from position B to position H. The continuous movement is provided, as explained above and as will be further explained below with reference to Figs. 8 an 9, by means of a chain drive, while the intermittent movement is provided by the aid of means which will be described below with reference to Fig. 7.

The lower left-hand corner of Fig. 7 shows the upper part of the triangular frame 55 of a frame, from which frame 55 two pins 50 extend, which, in a manner to be described below with reference to Figs. 8 and 9, are suspended in parts being guided in the upper rail 18. This rail 18 and the chain 21 together with the chain housing 22 are not shown in Fig. 7 for reasons of clarity. As mentioned above and as will be explained in greater detail below with reference to Fig. 8, the frames 17 are decoupled from the continuously working chain drive from the position A to a position corresponding to the middle of the semicircular rail section 26 of the lower rail 19.

As is evident from Fig. 1, a total of six frames are arranged consecutively in positions B, C, D, E, F and G. Form the positions B-G and after carrying out the operations described above at the respective positions the frames have to be forwarded to the next position, i.e. the frame in position B has to be forwarded to position C, etx., which means that the frame s in positions B-G as a whole have to be forwarded to the positions C-H. Therefore, the forwarding is preferably carried out in one move by means of a single carrier device which, as shown in Fig. 7, is a connectable and disconnectable carrier device.

The carrier device has a driving part 170 which is suspended in swivel arms 171 the upper ends of which are mounted on shafts which are stationary in relation to the chain housing 22 and rail 18 of the apparatus, as is evident from Fig. 1. The driving part 170 has recesses 173 for receiving and engaging with the pins 50 of said total of six frames which are to be moved as a whole from positions B-G to positions C-H. Fluid driving cylinders 174 serve to connect and disconnect the driving part 170 with the pins 50 and to swing the driving part 170 of the swivel arms 171 about the shafts 172. Consequently, the fluid driving cylinders 174 may move the driving part 170 in directions indicated by arrows 176 and 178. A fluid driving cylinder 179 serves to provide the above total movement of said total of six frames, which fluid driving cylinder 179 may drive the driving part 170 and thus the pins 50 into engagement with the driving part 170 and the associated frames with carcasses in a direction indicated by the arrow 177 and further return the driving part 170 to its starting position, i.e. in a direction indicated by the arrow 176. The driving pat 170 is consequently forwarded along the rectangular path indicated by arrows 175-178 by the driving cylinders 174 and by the driving cylinder 179. To monitor the movement of the driving part 170 in the directions indicated by the arrows 175 and 177 and further to monitor the movement of the driving part 170 and thus the movement of frames and the carcasses arranged thereon in the direction indicated by the arrow 177, a position detector device 180 is mounted on the upper side of the driving part 170, which device 180 may be a capacitive or preferably inductive proximity detector. By the provision of the detector 180 which generates and supplies a detector signal to the computer of the apparatus, not only the position of the driving part 170 may be monitored, but the movement of the driving part and thus the movement of the frames and the carcasses fixed thereon, which may be of very varying weight and thus relative to the force generating cylinder 179 offers a varying mass, is controlled in a given manner, especially according to a given acceleration/deceleration curve and within permissible maximum acceleration and deceleration limits. During its movement in the direction indicated by the arrow 177 the driving part 170 is guided relative to a stationary guide part 181, which is fastened to the upper rail 18 of the apparatus, as is evident from Fig. 1, by means of guide rolls 182.

For maintaining the frames in positions B-G a frame fixation part 183 is mounted on the opposite side of the chain 21 and the chain housing 22, which frame fixation part 183 has claw-shaped parts 184 for engagement with the frame pins 50. Like the driving part 170 the frame fixation part 183 is suspended swingably in the swivel arm 185 which corresponds to the swivel arm 171 described above, and which is in its turn fastened stationarily to shafts 186 which correspond to the shafts 172 described above. Fluid driving cylinders 187 serve to provide a swinging movement along directions indicated by a double arrow 188. The swinging movement of the frame fixation part 183 and thus the engagement of the claw-shaped parts 184 with the pins 50 is synchronized with the movement of the driving part 170 by the computer of the apparatus in such a manner that the pins 50 are always held fixedly either by the driving part 170 or by the claw-shaped parts 184 of the frame fixation part 183.

Fig. 8 shows a detail of a part of the chain conveyor shown in Fig. 1. The chain conveyor drives the carriers along the path defined by the chain housing 22 at a constant rate. As exaplained above this continuously working chain conveyor and the associated carriers 20, however, merely serve to forward frames which have been ejected from position H by means of the frame ejector swivel arm assembly 31 to the position A shown in Fig. 1, in which position the frame is waiting to be forwarded to position B for receiving a carcass. From position A to a position after the position H, the carriers are consequently disconnected from engagement with the frames 17. To provide this disconnection, a rail 190 is provided on the part of the path defined by the rail 18 on which part the carriers 20 are not to engage with the frame, and which rail 190 lifts the carriers so that they cannot engage with the associated engagement parts on the frames. The rail 190 is also shown in Fig. 1.

Fig. 9 shows another detail of the continuously working chain conveyor and further the suspension of the frames 17 in the rail 18. Thus, Fig. 9 shows two pins 50 of the rear end, seen in the direction of movement, of the triangular frame 55 of the frame 17, and the front end of the triangular frame of a subsequent frame 17. The pins 50 are suspended in U-shaped brackets 192 which are in their turn fastened to associated blocks 193 which are fastened each by two pins 194 to a carriage 195 enclosed in the rail 18, which carriage 195 has idle rollers or wheels 196. As it appears from Figs. 8 and 9, the rail 18 consists of two rail halves which define an upper gide groove and a lower guide channel through which lower guide channel the pins 194 extend. Through said upper guide groove an engagement pin 191 extends from the carriage 195 connected with the above mentioned pin 50, and this engagement pin 191 is adapted to be caught by the claw-shaped outer carrier end of the carrier 20 when the carrier 20 is not, as shown in Figs. 8 and 9, lifted to positions in which the carrier cannot engage with the pin 191. From the carriage 195 associated with the pin 50 a cam-shaped disconnecting part 197 extends upwards through the upper guide groove, which disconnecting part 197 disconnects the carrier 20 from engagement with the pin 191 when two consecutive frames have been moved into positions immediately after each other, which corresponds to a situation in which the frame which is shown in position I in Fig. 1 has been forwarded to a position immediately after position A, in which situation the cam-shaped part 197 of the frame which is in position A disconnects the carrier forwarding said frame from its engagment with the pin-shaped part 191 of this frame.

Apart from the purely mechanical disconnection mechanism, detector means are prefereably provided in the apparatus as is evident from Fig. 1, which detector means serve to monitor that when a carcass is introduced into the intermittently working part of the apparatus by means of the telescopically displaceable claw 95, a frame has been forwarded to position B, the computer of the apparatus being programmed to prevent introduction of a carcass into the intermittently working part of the apparatus from a position corresponding to the position in which the carcass 39 is shown in Fig. 1, unless a frame is present in position B. Correspondingly, further detectors are provided to monitor the correct operation of the apparatus at other positions in the apparatus.

Fig. 10 schematically shows the total of nine branding assemblies which are mounted on the stamp support 84, which branding assemblies are controlled by the computer of the apparatus in the manner described above so that when a carcass is in position G in the apparatus shown in Fig. 1, the assemblies produce a branding on selected parts of the carcass in question in accordacne with the measurements and quality determinations alredy made for the carcass in question.

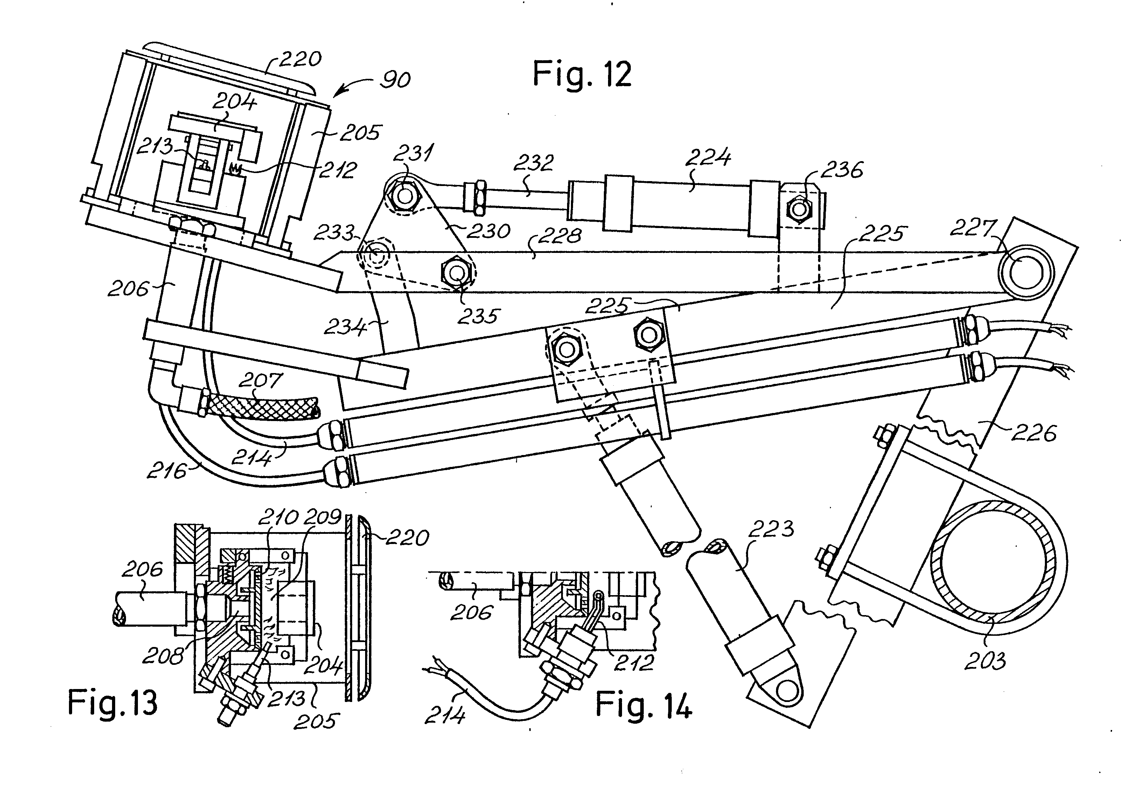

Apart from the above mentioned branding assemblies 86, 87, 88, 89, 90, 91 and 92, Fig. 10 shows further two branding assemblies designated 201 and 202. The branding assemblies 86, 88, 91, 201 and 202 are adapted to carry out a classification and part classification marking of the individual carcass controlled by the computer of the apparatus in accordance with the quality testing made in the probe measuring station shown in Fig. 6. The branding assemblies 86, 88, 91 and 201 are thus adapted to provide a part classification marking, i.e. adapted to provide a marking corresponding to one of the following symbols: [ A1], [X], [Z], [H], [G] or [W], while the branding assembly 202 is adapted to provide a marking corresponding to a sales class, i.e. adapted to provide a marking of one of the following symbols; [A1], A1, A or B. The remaining four branding assemblies, i.e. the branding assemblies 87, 89, 90 and 92 are so-called EØF (EEC) branding assemblies serving to provide a date stamp, a stamp of origin, i.e. indication of the slaughterhouse from which the carcass in question originates, and further a marking identifying the veterinary control of the slaughterhouse. The stamps serving to provide one and the same marking or branding (EEC marking) on all carcasses are of the type shown in Fig. 10 (the branding assemblies 87, 89 and 92) or of the type shown in Fig. 12 (the branding assembly 90). The difference between the branding assemblies shown in Figs. 11 and 12 is merely that the movement of the branding assembly shown in Fig. 11 is exclusively a linear reciprocating movement, while the branding assembly shown in Fig. 12 is adapted to carry out a circular or a swinging movement. As will be immediately understood, this difference is determined by the actual branding place on the carcass and of the position of the branding assembly in an inactivated condition, in which inactivated condition the carcass has to be able to pass to and from the position G, and in an activated condition, in which activated condition the branding element of the branding assembly is brought into contact with the carcass.

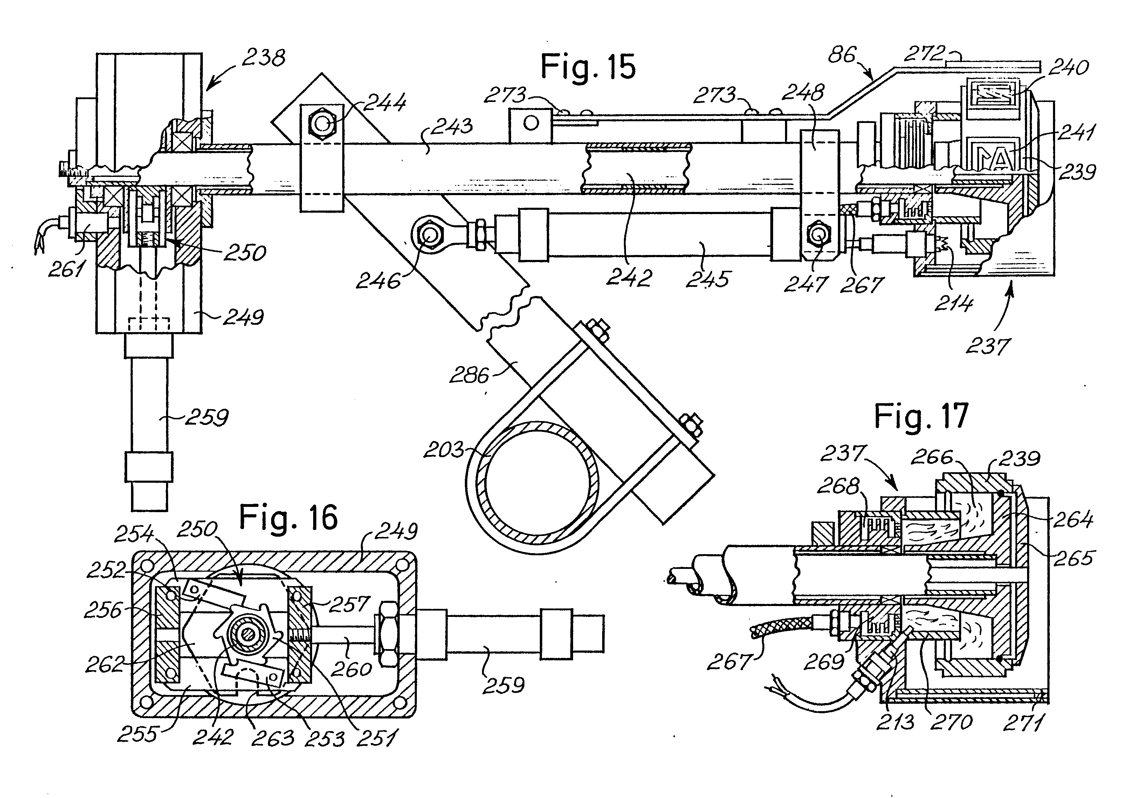

The branding assemblies 86, 88, 91, 201 and 202 constitute branding assemblies implemented in accordance with a particular aspect of the present invention and are, as already mentioned, part classification branding assemblies (the branding assemblies 86, 88, 91 and 201) and branding assemblies for marking of a sales class (the branding assembly 202), i.e. branding assemblies for providing a brand controlled by the computer of the apparatus of the invention identifying an examination or classification carried out for the carcass in question and thus a quality determiantion carried out for the carcass in question or, as mentioned above, an individual part of the carcass. These branding assemblies will be explained in greater detail below with reference to Figs. 15-17.

The branding assemblies 86-92, 201 and 202 shown in Fig. 10 are fastened to the branding assembly support 84 by means of a cylindrical column 203 on which the individual branding assemblies are fastened stationarily. Fig. 11 shows in greater detail the branding assembly 87 which is identical to the branding assemblies 89 and 92 shown in Fig. 10. Fig. 10 futher shows a pneumatic cylinder 284 which is adapted to position the branding assemblies 91 and 92 arranged on a bushing 285 displaceable relative to the cylindrical column 203 in a correct position relative to the carcass 40 controlled by the computer of the apparatus on the basis of the determination carried out previously in the apparatus of anatomical positions characteristic of the carcass in question in accordance with the principles of the present invention.

Fig. 12 shows a branding assembly 90 which, as mentioned above, is substantially of the same structure as the branding assembly 87 shown in Fig. 11, which is in its turn identical to the branding assemblies 89 and 92 shown in Fig. 10, except for the difference mentioned above in respect of movement relative to the carcass to be branded. The branding assembly 90 and the corresponding branding assemblies 87, 89 and 92 are further substantially of the construction described in the Applicants' Danish patent application No. 5025/81, which patent application is hereby incorporated by reference. The branding assembly 90 has a front part which is shown in detail in Fig. 13 and in which a branding element or branding block 204 is enclosed in a reciprocatingly movable cylindrical shield 205, which branding block 204 is exchangeable and is heated by a gas flame which is produced in the manner shown in Fig. 13 in a gas combustion chamber 209 behind the branding block 204 by supply of a combustible gas/air mixture via a pipe 206, and via a gas hose 207 in the assembly shown in Fig. 12. The combustible gas/air mixture is passed from the pipe 206 to a chamber 208 which is defined relative to the above gas combustion chamber 209 by a perforated plate 210. The combustible gas/air mixture is ignited by means of a incandescence ignition 212 which is shown in detail in Fig. 14 and in Fig. 12. The combustion of the combustible gas/air mixture is monitored by means of a flame control electrode 213 which is shown in Figs. 11, 12 and 13.

The incandescene ignition 212 and the flame control electrode 213 are connected via cables 214 and 216, respectively, to the computer of the apparatus which computer initially ignites the combustible gas/air mixture in the gas combustion chamber 209 or, if the combustion of this combustible gas/air mixture has been interrupted intentionally or unintentionally, reignites the combustible gas/air mixture and further monitors the combustion by means of the flame control electrode 213 and also controls the supply of gas from an external gas supply source, not shown on the drawings, via valve means which are not shown on the drawings either.

The above cylindrical heat shield 205 is mounted encircling the branding block 204 which, as mentioned above, is exchangeable and which may be composed of several individual elements which together serve to provide, for example, a date mark, a mark of origin, i.e. an EEC mark, and serves to restrict the heat radiation from the heated branding elements, i.e. from the branding block 204, of the branding assemblies in the positions shown in Figs. 11 and 13 for the branding assemblies 87 and 90, shown in Figs. 11 and 12, and thus to reduce the energy or gas consumption. An abutment member 220 is provided at the front of this heat shield 205 which abutment member 220 is annular and, as is evident from Figs, 11, 12 and 13, has an arched cross-sectional shape, and which abutment member 220, when the branding assembly as will be explained below is passed towards the carcass to be branded serves to stretch or tighten the surface of the rind at the place where the branding block 204 thereafter carries out the actual branding. This stretching or tightening of the surface of the rind has proved to be of importance for obtaining a good branding result. As already indicated above, the branding is performed by the branding block 204 being passed out through the central opening of the annular abutment member 220, or more correctly by the cylindrical heat shield 205 with annular abutment member 220 fastened thereto being retracted relative to the branding block 204.. The branding assemblies shown in Figs. 11 and12 thus are proviced with means for producing this mutual movement betwen the heat shield 205 and the branding block 204 and further means for passing the front part of the branding assembly as a whole, comprising the above mentioned parts, towards the carcass to be branded.

In the embodiment shown in fig. 11 the pipe 206 is fastened to a plate 221, and the pipe and the plate are displaced relative to a supporting pipe 222 by the activation of a fluid pressure cylinder 223. The plate 221 also supports another fluid pressure cylinder 224 which, by activation, retracts the heat shield 205 and the annular abutment member 220 relative to the branding block 204 and thus provides the relative displacement between the branding block 204 and the heat shield 205 together with the abutment member 220. The fluid pressure cylinders 223 and 224 are controlled by the computer of the apparatus, and the movements of the branding assembly and of the heat shield 205 relative to the branding block 204 are monitored by means of motion sensors such as capacitive or, preferably inductive sensors generating and supplying signals to the computer of the apparatus for the provision of a reliable control of the movements described above.

In the embodiment shown in Fig. 11, the movements of the branding assembly 87 and of the heat shield 205 and the abutment member 220 relative to the branding block 204 are linear reciprocating movements while the corresponding movements of the branding assembly 90 shown in Fig. 12 are swinging movements which, as mentioned above, are necessary in connection with branding at certain positions of the carcass 40 shown in Fig. 10. Like in the embodiment shown in Fig. 11, the movements are produced by means of the fluid pressure cylinders 223 and 224. In the embodiment shown in Fig. 12 the branding assembly 90 is mounted on a swivel arm 225 which is swingably mounted in a swivel bolt connection 227 on a beam 226 bolted to the cylindrical column 203. Activation of the fluid pressure cylinder 223, which is fastened to the swivel arm 225 and to the beam 226, produces a swinging of the branding assembly 90 around the axis of the swivel bolt connection 227. The heat shield 205 is also mounted swingably about the axis of the swivel bolt connection 227 on an arm 228, this arm 228 is capable, by the activation of the fluid pressure cylinder 224, of producing a relative rotation relative to the swivel arm 225 via a three-point connection 230 which has a swivel bolt connection 231 connected to the piston 232 of the fluid pressure cylinder 224, a swivel bolt connection 233 firmly connected to the swivel arm 225 via an angular arm 234, and a third swivel bolt connection 235 connected to the swivel arm 228. The end of the fluid pressure cylinder 224 opposite to the piston 232 is rigidly connected to the swivel arm 228 by a swivel bolt connection 236. By the activation of the fluid pressure cylinder 224 in such a manner that the piston 232 of this fluid pressure cylinder is passed into the fluid pressure cylinder, the arm between the swivel bolt connections 231 and 236 is shortened, and as a result of the fixation of the three-point connection 230 in the swivel bolt connection 233 to the swivel arm 225, the swivel bolt connection 235 performs a rotation clock-wise about this firm swivel bolt connection 233, which results in that the heat shield 205 is swung in an anti-clockwise direction and thus is retracted relative to the branding block 204 and thus uncovers the branding block.

The activation of the fluid pressure cylinders 223 and 224 is also controlled by the computer of the apparatus in the embodiment shown in Fig. 12, and the movement of the branding assembly 90 and the heat shield 205 is also monitored by the computer by means of position detectors such as capacitive or preferably inductive sensors. In the environment to be found in a slaughterhouse, it is, however, most often preferred to us inductive sensors as capacitive sensors cannot under all conditions be expected to produce reliable and unambiguous detections as a result of humidity and grease films.

The branding assembly shown in fig. 15 is, as mentioned above, a part classification branding assembly which is mounted swingably on a beam 286 bolted firmly to the pipe 203. A first end 237 of the branding assembly 86 contains the branding element 239 of the branding assembly, which branding assembly is a cylindrical body on the outer side of which a total of six part classification branding elements are arranged of which assembly two elements designated 240 and 241 are shown in Fig. 15. The branding element 239 is positioned by means of positioning means mounted at another end 238 of the branding assembly 86 opposite to the said first end 237 of the branding assembly. The branding element 239 is rotatably mounted on a pipe 242 which is enclosed in and is rotatable relative to an enclosing pipe 243, and which is connected to the positioning means arranged at the other end 238. The said pipe 243 constitutes a swivel arm relative to the beam 286 and is connected thereto in a swivel bolt connection 244, the swinging movement of the pipe 243 relative to the beam 286 around the axis of the swivel bolt connection 244 is provided by a fluid pressure cylinder 245, one end of which is connected to the beam 286 via a swivel bolt connection 246, and the other end of which is connected to the pipe 243 via a swivel bolt connection 247 and a flange 248 fastened to the pipe 243. As will be understood, a shortening or lengthening of the distance between the swivel bolt connections 246 and 247 by a shortening and a lengthening, respectively, of the length of the fluid pressure cylinde 245 by activation of this fluid pressure cylinder produces a swinging of the pipe 243 around the axis of the swivel bolt connection 244 clockwise and anti-clockwise, respectively. This swinging movement is controlled by the computer of the apparatus and is further monitored by means of motion or position sensors such as inductive sensors of the same type as the inductive sensors mentioned above and used for monitoring of the movements of the branding assemblies shown in Figs. 11 and 12.

The turning of the branding element and the positioning of the six part classification branding elements of this branding element 239 such as the part classification branding elements 240 and 241 shown in Fig. 15 are provided by means of positioning means which, as already mentioned above, are mounted at said other end 238 of the branding assembly 86 and are designated 250 as a whole. The positioning means 250 are shown in greater detail in Fig. 16 and are contained in a housing 249. The positioning means 250 are, as already mentioned above, connected to the branding element 239 via the pipe 242, this separation of the branding element 239 and the positioning means 250 via the pipe 242 serves to restrict the transfer of heat from the heated branding element 239 to the positioning means 250 and thus to eliminate any harmful or even destructive thermal action from the hot branding element 239 on the positioning means 250. The pipe 242 may be made from a material with a relatively low thermal conductivity, for example stainless steel, and/or for restricting the heat transmission of heat from the branding element 239 to the positioning means 250 via the pipe 242 cooling may be provided to the pipe 242, as cooling liquid or cooling air may be passed through the pipe 242 or into the space between the pipes 242 and 243 or to a part of the space defined between these pipes, and as a closed cooling circuit may be provided in connection with such liquid or air cooling, in which circuit the circulating cooling medium may be cooled in heat exchanger means.

As shown in Fig. 16, the positioning means 250 comprises a ratchet wheel 251 mounted on the pipe 242 and adapted to cooperate with two block-shaped bodies 252 and 253 which are mounted on two beam-shaped bodies 254 and 255, respectively, which beam-shaped bodies are interconnected via two block-shaped bodies 256 and 257 and together with these block-shaped bodies form a rectangular frame which may be reciprocated in the housing 249 perpendicularly to the longitudinal axis of the pipe 242. The reciprocal displacement of the frame formed by the bodies 254-257 is generated by means of a fluid pressure cylinder 259, the outer end of a movalbe piston 260 of which is screwed into an internal thread of the body 257. By displacement of the piston 260 of the cylinder 259 in teh cylinder 259, the frame formed by the bodes 254-257 is displaced to the right in Fig. 16, whereby the block-shaped body 252 acts on the ratchet wheel 251 which thus turns an angle defined by the movement or travel of the frame formed by the bodies 254-257 from the position shown in Fig. 16 to the right, driven by the piston 260 of the fluid pressure cylinder 259. In the return of the frame formed by the bodies 254-257 from this position - displaced towards the right in Fig. 16 - to the position shown in Fig. 16, the block-shaped body 253 acts on the ratchet wheel 251 in the manner just described for the block-shaped body 252. In the embodiment shown in Figs. 15-17 the reciprocating movement of the piston 260 driven by the fluid pressure cylinder 259 is controlled in exactly such a manner that this reciprocating movement causes a joint travel of the frame formed by the bodies 254-257, which travel of the block-shaped bodies 252 and 253 is converted into a rotation of the ratchet wheel and thus a rotation of the pipe 242 of 60°. Consequently, the ratchet wheel 251 has a total of six peripheral projections of which each corresponds to a corect positioning of a part classification branding element of the branding element 239. The current position of the branding element 239 is monitored by means of an inductive sensor 261 shown in Fig. 15, which sensor cooperates with a hexagonal detector disc 262 which is rigidly connected to the pipe 242, and which has a reference recess 263.

Fig. 17 shows a sectional view through the first end 237 of the branding assembly 86. The annular branding element 239 is mounted on the outside of a body 264 which is mounted at the end of the pipe 242. At the end of the body 264 and further at the end of the annular body 239 a disc-shaped body 265 is mounted. These bodies 264 and 265 are, like the branding element 239, of a high-temperature resistant metal and in mutually thermally conductive connection. As appears from Fig. 17, an inner annular chamber 266 is formed between the branding element 239 and the body 264, which chamber 266 constitutes a gas combustion chamber. The gas combusiton chamber 266 is supplied with a combustible gas/air mixture from a supply conduit 267 via a chamber 268 in which a body 269 is mounted and defines a labyrinth-shaped passage in the chamber 268. From the chamber 268 the gas is passed via perforations in a perforated plate into the chamber 266, into which chamber 266 a wall 270 extends. In the chamber defined between the wall 270 and the body 264, the above mentioned flame control electrode 213 is mounted.

Encircling part of the annular branding element 239, a heat shield 271 is mounted, which shield 271 serves the same purpose as the heat shield 205 described above the reference to Figs. 11-13. It is, however, to be noted that the heat shield 271 does not cover the end of the branding assembly 86. In the space between the inner side of the heat shield 271 and the outer side of the wall 270 the above desciribed incandescence ignition 214 takes place. For providing a screening of the rind at the place on the carcass where a brand is to be provided, a spring-biassed abutment means 272 having a rectangular opening is mounted in alignment with the part of the outer cylinder surface of the branding element 239, which part is uncovered relative to the heat shield 271. The abutment means 272 thus primarily serves the primary purpose of providing a screening of the individual branding element relative to the adjacent branding elements, and the secondary purpose of providing the above stretching or tightening of the rind. This abutment means 272 is also shown in Fig. 10. The abutment means 272 is fastened to the outer side of the pipe 243 by means of bolts 273.

Although the apparatus has been described with reference to a specific embodiment in which the carcasses are moved through specific stations corresponding to the positons B-H described above by means of an intermittently working carrier structure and fixed on frames which, by means of a continuosuly working conveyor, are returned to an input stations, it is understood that numerous modifications are possible within the scope of the invention. Thus, the individual frame may be provided with its own driving motor which may, for example, be an electric motor. and which is controlled individually by the computer and is monitored by position, velocity-and acceleration detectors so that the infividual frame with associated carcass is passed through the stations of the apparatus by the associated driving motor in a safe and reliable manner within prescribed positioning, velocity and acceleration limits. The determinations of dimensions which are made in the apparatus described above by means of the devices described with reference to Figs. 2, 4, and 5, may futher possibly be carried out in another manner. Thus it is believed that the computer of the apparatus may be connected to or constituted a pattern recognition computer which may provide data representing characteristic anatomical data for the individual carcass by processing of a television image in a manner known per se .

Furthermore, instead of the positioning means of the branding assemblies described above, any electric, hydraulic or pneumatic driving means, for example a step motor, may be used, and instead of six part classification branding elements any number of individual branding elements may be provided on the annular branding element 239. These individual branding elements only have to have such an extent that it is ensured that a branding is carried out with only a single one of these individual branding elements, which is further ensured by the abutment means 272 described above. Such alternatives are also intended to be comprised by the present invention as it is defined in the following claims.

## Claims

1. A method of treatment or examination of carcasses of slaughter animals, especially hogs or porkers, in at least one treatment or examination station, in which method the carcasses are moved substantially continuously and consecutively by a first transport system, such as a sliding rail transport system, along a given path, the carcasses being passed to and from the treatment or examination station by the first transport system, and in which method the treatment or examination is carried out in the treatment or examination station by means of treatment or examination means adjustable relative to the individual carcass, **characterized** in that the carcasses are forwarded through the treatment or examination station by means of a second transport system which is an intermittently working transport system, and which defines a substantially linear path of transportation, at which the treatment or examination means are arranged, that carcasses are transferred from the first transport system to the second transport system prior to the treatment or examination station, and that after transportation through the treatment or examination station by means of the second transport system the carcasses are transferred therefrom to the first transport system.

2. A method according to claim 1, **characterized** in that the carcasses are transferred from the first transport system to an input station arranged prior to the treatment or examination station of the second transport system, and that prior to or at the input station and/or at a registration station arranged between the input station and the treatment or examination station a registration is carried out of a measure of at least one outer dimension of the individual carcass, and that said measure is converted, on the basis of empirical data for the relevant type of slaughter animal, into a control signal for controlling the positioning of the treatment or examination means relative to the carcass in question.

3. Method according to claim 2, **characterized** in that the carcasses are forwarded in the first and the second transport system suspended by their hind legs, that a registration is carried out of a measure for the total length of the individual carcass, that the registration of the measure of the total length is preferably carried out by means of a movable plate comparing an associated position detecting device, the movable plate being adapted to be brough into contact with the bottommost part of the individual carcass, and/or that a registration of a measure of the positioning of at least one extremity of the individual carcass, especially the position of one foreleg, is carried out at the registration station, and preferably that a further registration is carried out of a measure for the position of the collarbone of the individual carcass.

4. A method according to claim 2 or 3, **characterized** in that the conversion of said measure or measures into said control signal is carried out by conversion in accordance with a linear algorithm.

5. A method according to any of the claims 2-4, **characterized** in that a registration is further carried out of the weight of the individual carcass.

6. A method according to any of the preceding claims, **characterized** in that a sampling, a meat quality test, a marking and/or a cutting up is carried out in the treatment or examination station.

7. A method according to any of the preceding claims, **characterized** in that the carcass are forwarded in the second transport system suspended in frames for fixation of the carcasses, and that the frames are forwarded in a closed path of which said substantially linear path of transportation constitutes a part.

8. An apparatus for treatment or examination of carcasses of slaughter animals, especially hogs, and comprising at least one treatment or examination station and a first transport system, in which the carcasses are forwarded substantially continuously and consecutively along a given path, the carcasses being passed to and from the treatment or examination station by the first transport system, in which treatment or examination station the treatment or examination is carried out by means of treatment or examination means adjustable relative to the individual carcass, **characterized** in that the apparatus comprises a second transport system for forwarding the carcasses through the treatment or examination station, that the second transport system is an intermittently working transport system defining a substantially linear path of transportation at which the treatment or examination means are arranged, and that the first and the second transport systems are adapted to transfer the individual carcass from the first transport system to the second transport system prior to the treatment or examination station and to transfer the individual carcass from the second transport system and to the first transport system after transportation through the treatment or examintion station.

9. An apparatus according to claim 8, **characterized** in that the second transport system of the apparatus comprises an input station arranged prior to the treatment or examination station, to which input station the carcasses are transferred from the first transport system, and that the apparatus comprises means arranged prior to or at the input station and/or at a registration station arranged between the input station and the treatment or examination station for registration of a measure of at least one outer dimension of the individual carcass, and a central control unit in which empirical data for the relevant type of slaughter animals are stored, and in which the registered measure is converted, on the basis of these empirical data, into a control signal for controlling the positioning of the treatment or examination means relative to the carcass in question.

10. An apparatus according to claim 9, **characterized** in that the apparatus comprises means for forwarding the carcasses in the first and the second transport systems suspended by their hind legs, that the apparatus comprises a registration means arranged in the input station for registration of the total length of the individual carcass, that the length registration means arranged in the input station preferably consitituted by a movable plate comprising an associated position detecting device, the movable plate being adapted to be brought into contact with the bottommost part of the individual carcass, and/or that the apparatus comprises a registration means arranged in the registration station for registration of a measure of the position of at least one extremity of the individual carcass, especially the position of one foreleg, and preferably further comprises a registration means arranged in the registration station for registration of a measure of the position of the collarbone of the individual carcass.

11. An apparatus according to any of the claims 8-10, **characterized** in that the central device of the apparatus is adapted to carry out the conversion of said measure or measures into said control signal in accordance with a linear algorithm.

12. An apparatus according to any of the claims 8-11, **characterized** in that the apparatus further comprises scales for weighing the individual carcass and for generating a weight representing signal which is passed to the central control device of the apparatus.

13. An apparatus according to any of the claims 8-12, **characterized** in that the treatment or examination means arranged in the treatment or examination station comprises sampling means, quality testing means, marking means and/or cutting-up means.

14. An apparatus according to any of the claims 8-13, **characterized** in that the second transport system of the apparatus comprises a number of frames for fixation of carcasses and that the frames are adapted to be forwarded in a closed path of which said substantially linear path of transportation constitutes a part.

15. An apparatus according to claim 14, **characterized** in that the frames are suspended from a guide rail defining the closed path, and that the frames are further adapted to be guided in an underlying pilot rail defining the same closed path as the guide rail from which the frames are suspended.

16. An apparatus according to claim 14 or 15, **characterized** in that the second transport system of the apparatus is adapted to forward the frames in the part of the closed path which does not constitute said substantially linear path, in a continuous movement from an output station at which the carcasses are transferred from the second transport system to the first transport system to said input station.

17. An apparatus according to claim 16, **characterized** in that the second transport system of the apparatus comprises a continuously working conveyor for forwarding the frames from the output station to the input station, the frames and the conveyor being adapted to connect the frames with the conveyor at the output station and to disconnect the frames from the conveyor at the input station.

18. An apparatus according to claim 16 or 17, **characterized** in that the second transport system comprises a forwarding mechanism which may be connected with and disconnected from at least one frame and which is adapted to be driven by a fluid driving mechanism for providing the intermittent forwarding of a frame connected with the forwarding mechanism, the connectable and disconnectable forwarding mechanism preferably being a gripping mechanism which may be connected with and disconnected from said one frame.

19. An apparatus according to claim 17 or 18, **characterized** in that said continuosuly working conveyor is a chain conveyor having carrier members which are adapted to provide the connection of the frames to the conveyor at the output station and the disconnection of the frames from the conveyor at the input station, and that the carrier members are preferably constituted by hinged carrier fingers.

20. An apparatus according to any of the claims 16-19, **characterized** in that the apparatus comprises gripping means which are adapted to transfer a carcass from the first transport system to the second transport system and to arrange the carcass on a frame, and ejector means which are adapted to eject a carcass from its associated frame and to transfer the carcass from the second transport system to the first transport system.

21. An apparatus according to claim 20, **characterized** in that each frame is an openable frame, and that the apparatus comprises means for opening the frames at the input and output stations for introducing a carcass into a frame and for ejecting a carcass from a frame, respectively, and that the openable frame comprises gripping means for gripping around the head of a carcass, the gripping means preferably being adjustable gripping means adapted to be controlled by the central control unit of the apparatus in accordance with said measure or measures.

22. An apparatus according to claim 13, **characterized** in that said quality testing means comprise a number of probe units each comprising an insertion measuring probe, that the probe units are mounted on carriages the position of which may be adjusted individually controlled by the central control unit of the apparatus in accordance with said measure or measures, and that each carriage comprises devices for a joint movement of the probe units associated with the carriage in any direction in one plane.

23. An apparatus according to claim 22, **characterized** in that said devices comprise a first carriage system and a second carriage system mounted or said first carriage system, and that the carraige systems are adapted to move perpendicularly to each other driven by associated fluid driving mechanisms.

24. An apparatus according to claim 22 or 23, **characterized** in that each probe unit further comprises a cylinder with a through-going piston rod, one end of which serves as an insertion probe and comprises means for measuring light reflection, and the other end of which is connected to a position detecting device for detecting the travel or position of the piston rod.

25. An appratus for branding objects, especially carcasses of animals such as hogs, and having a branding iron comprising a rod-shaped or tubular member and branding elements arranged at a first end thereof, means for supporting the object to be branded, means for heating the branding elements of the branding iron and means for the providing a mutual displacement of the supporting means and the branding iron and, by this mutual displacement, for bringing the branding elements of the branding iron into contact with the object to be branded, **characterized** in that the branding elements of the branding iron comprise at least two sets of branding elements, that the branding iron further comprises positioning means arranged at the other end of the rod opposite to said first end, for changing the branding iron between a number of positions corresponding to the number of sets of branding elements, in which positions the respective sets of branding elements are brough into contact with the object to be branded by the provision of said mutual displacement of the supporting means and the branding iron, and that the rod-shaped or tubular member of the branding iron has such a length and/or is cooled in such a manner that transmission of heat from the branding elements heated by means of the heating means to the positioning means of the branding iron is restricted to such an extent that a thermal action harmful or destructive for the positioning means is avoided.

26. An apparatus according to claim 25, **characterized** in that sets of branding elements are arranged in such a manner that a displacement of the branding iron in the lonigtudinal direction of the rod and/or a rotation of the branding iron around the axis of the rod changes the branding iron between said positions, and that the positioning means are adapted to produce a linear displacement of the branding iron in the longitudinal direction of the rod and/or a rotation of the branding iron around the axis of the rod.

27. An apparatus according to claim 26, **characterized** in that the branding elements are arranged peripherally and at the same mutual peripheral distance around the rod-shaped or tubular member of the branding iron, and that the positioning means are adapted to produce a step-wise rotation of the branding iron around the axis of the rod corresponding to said peripheral distance.

28. An apparatus according to claim 27, **characterized** in that the branding iron comprises a total of six branding elements each having a peripheral extent corresponding to an angle of 60°, and that the positioning means comprise a ratchet device adapted to permit a rotation of the branding iron in one direction and to prevent rotation of the branding iron in the opposite direction past said positions.

29. An apparatus according to claim 28, **characterized** in that the positioning means comprise a cylinder cooperating with said ratchet device for providing the step-wise rotation of the branding iron.

30. An apparatus according to any of the claims 25-29. **characterized** in that said heating means comprise gas burner means, flame monitoring means, and spark ignition means or preferably incandescence ignition means.

31. An apparatus according to any of the claims 25-30, **characterized** in that the branding elements of the branding iron are at least partially enclosed in a heat insulating shield.

32. An apparatus according to any of the claims 25-31, **characterized** in that the branding iron comprises a spring-biassed abutment member for abutment against the surface of the object to be branded, immediately before a set of branding elements is brought into contact with said object by said mutual displacement of the supporting means and the branding iron.

33. A branding iron for use in an apparatus according to any of the claims 25-32, **characterized** in that the branding iron comprises any of the characteristics of the branding iron of the apparatus indicated in claims 25-32.

# Fig. 1

0 273 371

Fig. 2

Fig. 3

Fig. 4

Fig. 5

Fig. 6

Fig. 8

Fig. 9

Fig. 7

Fig. 10

Fig. 11

Fig. 12

Fig. 13

Fig. 14

0 273 371

Fig. 15

238

244

273

273

248

86

272

240

241

239

243

242

245

247

267

214

237

246

286

203

261

250

249

259

0 273 371

Fig. 17

237

266

239

268

264

265

Fig. 16

254

250

249

252

256

257

262

260

267

269

255

242

263

253

251

259

213

270

271